(19) Europäisches Patentamt
European Patent Office
Office européen des brevets

(11) EP 4 306 548 A1

(12) # EUROPEAN PATENT APPLICATION

(43) Date of publication:
**17.01.2024 Bulletin 2024/03**

(21) Application number: **22184567.0**

(22) Date of filing: **13.07.2022**

(51) International Patent Classification (IPC):
***C08B 37/02*** (2006.01) ***A61K 47/36*** (2006.01)
***A61L 27/52*** (2006.01) ***C08J 3/075*** (2006.01)
***C08L 5/02*** (2006.01) ***C08B 37/08*** (2006.01)

(52) Cooperative Patent Classification (CPC):
(C-Sets available)
**C08B 37/0021; A61L 27/26; A61L 27/3804;**
**A61L 27/50; A61L 27/52; A61L 27/54;**
**C08B 37/0072; C08J 3/075; C08L 5/02;** A61K 9/06;
A61K 47/36; A61L 2300/43; A61L 2400/06;
C08J 2305/02; C08J 2305/08; (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Adocia**
**69003 Lyon (FR)**

(72) Inventors:
- **GEISSLER, Alexandre**
**69330 Meyzieu (FR)**
- **LAURENT, Nicolas**
**01700 Miribel (FR)**
- **PLANCQ, Baptiste**
**01700 Saint Maurice de Beynost (FR)**
- **SOULA, Gérard**
**69330 Meyzieu (FR)**
- **ELOY, Marie-Rose**
**69006 Lyon (FR)**

(74) Representative: **Tripoz, Inès**
**Cabinet Tripoz**
**Le Pôle Sud**
**22 rue Seguin**
**69002 Lyon (FR)**

(54) **HYDROGELS FOR CELL THERAPY**

(57) The invention concerns an hydrogel comprising:
- biological cells,
- a non crosslinked hyaluronic acid in the form of a solution, and
- a crosslinked dextran polymer Dx bearing anionic groups wherein the at least divalent radical $L(-)_i$ is covalently bound to the dextran polymer backbone with i W radicals, wherein,
- $L(-)_i$ is a linear or branched polyether
- i is the valence of L and the number of W radicals bound to the dextran polymer and is an integer comprised from 2 to 8 ($2 \leq i \leq 8$),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

It also concerns the therapeutic use of the hydrogel according to claim 1, for treating a disorder or disease in a mammal wherein the disorder or disease is due to lack or malfunction of endocrine function of pancreas organ.

It also concerns an implant comprising the hydrogel according to any one of claims 1 to 2.

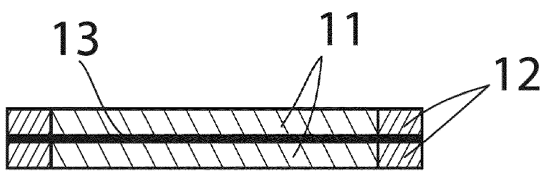


FIGURE 1

EP 4 306 548 A1

(52) Cooperative Patent Classification (CPC): (Cont.)
C08J 2371/02; C08J 2405/02; C08J 2405/08;
C08J 2471/02

C-Sets
**A61L 27/26, C08L 5/02;**
**A61L 27/26, C08L 5/08;**
**C08L 5/02, C08L 5/08**

## Description

The domain of the invention is therapy, in particular cell therapy. More particularly the invention is about an implant comprising a hydrogel which may incorporate:

- active principles, such as peptides, hormones or proteins, or
- secreting cells, which may be cells secreting peptides or hormones.

The aim is to prevent, treat or cure disease. In particular, this could allow the prevention and/or treatment of chronic diseases by replacing totally or in part the function of naturally occurring cells which are deficient in the patients. The invention is also about a crosslinked polymer, its precursors, a process for obtaining the crosslinked polymer and a process for obtaining a hydrogel, in particular a hydrogel containing cells.

The cells may be isolated or aggregated and may be of one type or of different types.

Hydrogels can be used in multiple systems like:

- scaffolds as controlled drug or active pharmaceutical ingredient release systems or
- scaffolds to be used as implantable device comprising cells.

Hydrogels comprise or consist of polymers that are crosslinked in a 3D network. They can either be natural or synthetic, homopolymers or copolymers. They have the ability to absorb and retain large amounts of water. This is known as the swelling of hydrogels.

In order to have a system which may be an implant able to deliver active principle on the long run, many features have to be obtained.

Among these features may be cited:

- a low degradability, in particular a low biodegradability, or no biodegradability, or a good in vivo stability, in order for the incorporated cells not to escape in the organism of the patient and the host cells not to penetrate in the implant,
- a good permselectivity, defined as the selective permeability toward biological elements according to their size or molecular weight, allowing a low, or even better no, immune response by isolating the incorporated cells, totally or in part, from the immune system of the host while allowing the passage of the active principle, for example a hormone, peptide or protein,
- a good mitigation of the foreign body response, or a good biocompatibility, in particular a low cytotoxicity and a good local tolerance,
- allowing the cells to have a high survival rate, such as having a good vascularisation close to the cells and sufficient flux of nutrients to the cells,
- allowing the cells to have a good functionality within the hydrogel.

In order to be used as controlled release systems or scaffolds for cells, hydrogels must have particular characteristics so as to exhibit all or part of the desired properties such as disclosed above as well as good mechanical and rheological properties.

Among the rheological and mechanical properties that are of high interest for the hydrogel may be cited:

- a good homogeneity, which can be linked to a good transparency or translucency,
- appropriate resistance and flexibility toward stress and strain mechanics, in particular when being handled and implanted, for example through laparoscopy,
- a defined mesh size to maximise oxygen and nutrients exchanges, controlled transport properties and permselectivity
- a good stability in vivo, i.e. resistance to hydrolytic, enzymatic or oxidative degradation.

Among parameters which can give indications on the rheological and mechanical desired properties may be cited:

- tan $\delta$ (called loss tangent) which gives indication on mechanical properties,
- G', which gives indication on the elastic modulus (stiffness), and on the mesh size,
- compression and/or traction deformation at break, which gives indication on the elasticity and resistance of the hydrogel,
- swellability, which gives indications on the water content, dimensions and mechanical properties.

Among the problems to be solved is obtaining a hydrogel with properties allowing:

- a manipulation for implanting the hydrogel without breaking it, such as by laparoscopy, and/or
- a gel to remain in place after implantation, for example allowing the hydrogel not to get folded after implantation and/or to be immobilized relative to the tissue on which it is implanted.

Another problem to be dealt with relates to the sedimentation of the cells, or islets during the crosslinking leading to gelation.

The following prior art on hydrogel:

- Nestor Loper Mora et al, "evaluation of dextran(ethyleneglycol) hydrogel films for giant unilamellar lipid vesicle production and their application for the encapsulation of polymersome, Soft Matters, January 2017, Vol. 13, n°33, pp 5580-5585,
- Hanwei Zhang et al., "In situ gelable interpenetrating double network hydrogel formulated from binary components: thiolated chitosan and oxidized dextran", Biomacromolecules, 2011, Vol. 12, n°5, pp 1428-1437,
- Rongsheng Zhang et al., "A novel pH and ionic strength sensitive carboxymethyl dextran hydrogsel, Biomaterials, 2005, Vol. 26, n°22, pp 4677-4683, , and
- Taichi Ito et al., "Dextran-based in situ cross-linked injectable hydrogels to prevent peritoneal adhesions", Biomaterials, 2007, Vol. 28, n°23, pp 3418-3426, disclose hydrogels which are not able to solve technical problems as the hydrogels of the invention do.

In the prior art hydrogels comprising cells which are crosslinked are very often intended for allowing the growth of a cell object, for example 3D cell culture. This kind of application needs that the hydrogels could at the same time embed the starting cells and make space for the new cells obtained by outgrowth and/or proliferation. In order to comply with these two opposite characteristics, the solution is to have degradable, for example via cleavable bonds, hydrogels which are strong enough to embed the cells and which upon degradation make sufficient space for new cells. A method of choice to obtain this degradation is to have a peptidic structure within the crosslinked hydrogel, in particular at the level of the crosslinker in between the polymeric backbone.

This type of behavior is completely incompatible with the aim of the instant invention which is to obtain a long lasting crosslinked hydrogel encapsulating/embedding cells. In this case the hydrogel needs to have very little, or even better non-degradable, as this key feature will allow to keep the cells invisible from the immune system.

The underlying problem is solved by the provision of a gel that presents physicochemical properties to allow the manufacture of an implantable device and biocompatibility properties that allow the cells survival.

Moreover, and on the contrary to many prior arts, the invention allows the preparation of hydrogels which have tunable features, considering the precursors used and the way the crosslinking is performed. This can lead to hydrogels having a controlled incorporation and release of specific objects from the hydrogel.

The suitability of the hydrogel depends on its bulk structure, thus important parameters used to characterize the network structure of the hydrogel according to the invention are the polymer volume fraction in the swollen state, the molecular weight of the polymer chain between two neighboring cross-linking points, and the corresponding mesh size.

The problem is solved by the provision of a new Hydrogel comprising:

- biological cells,
- a non crosslinked hyaluronate in the form of a solution, and
- a crosslinked dextran polymer Dx bearing anionic groups wherein the at least divalent radical $L(-)_i$ is covalently bound to the dextran polymer backbone with i W radicals, wherein,
- $L(-)_i$ is a linear or branched polyether
- i is the valence of L and the number of W radicals bound to the dextran polymer and is an integer comprised from 2 to 8 ($2 \leq i \leq 8$),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

It also concerns and implant comprising the hydrogel of the invention.

The properties of this family of hydrogels are tunable and tailorable to the applications by choosing and adapting the cross-linking reaction conditions, the substitution degree and molecular weight of the dextrans and cross-linkers.

The applicant surprisingly found that the presence of hyaluronic acid in the crosslinking mixture helps improving a homogeneous repartition of the cells or islets in the hydrogel. In other words this decreases the effect of sedimentation of the cells or islets.

Furthermore, the presence of hyaluronic acid does impact negatively on the final hydrogel.

In an embodiment the hydrogel has a weight average molecular (Mw) ranging from 100 to 2 500 g/mol.

In an embodiment the hydrogel has Mw ranging from 250 to 2 500 g/mol.
In an embodiment the hydrogel has Mw ranging from 500 to 2 250 g/mol.
In an embodiment the hydrogel has a Mw ranging from 750 to 2 000 g/mol.
In an embodiment the hydrogel has a Mw ranging from 1 000 to 1 500 g/mol.
In an embodiment the concentration of hyaluronic acid in the hydrogel ranges from 0.5 to 30 mg/ml.
In an embodiment the concentration of hyaluronic acid in the hydrogel ranges from 0.5 to 20 mg/ml.
In an embodiment the concentration of hyaluronic acid in the hydrogel ranges from 0.5 to 10 mg/ml.
In an embodiment the concentration of hyaluronic acid in the hydrogel ranges from 0.5 to 5 mg/ml.
In an embodiment the concentration of hyaluronic acid in the hydrogel ranges from 0.75 to 2.5 mg/ml.
In an embodiment the concentration of hyaluronic acid in the hydrogel ranges from 1.0 to 1.5 mg/ml.
In an embodiment with a hyaluronic acid of Mw ranging from 1 000 to 2 000 g/mol, in particular of around 1 500 g/mol, its concentration is ranging from 0.5 to 2 mg/ml.
In an embodiment with a hyaluronic acid of 1 000 to 2 000 kD, in particular around 1 500 g/mol, its concentration is ranging from 1.0 to 1.5 mg/ml.
The cross-linked dextran polymer is a dextran polymer Dx bearing anionic groups wherein the at least divalent radical $L(-)_i$ is covalently bound to the dextran polymer backbone with i W radicals, wherein,

- $L(-)_i$ is a linear or branched polyether
- i is the valence of L and the number of W radicals bound to the dextran polymer and is an integer comprised from 2 to 8 ($2 \leq i \leq 8$),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

In an embodiment -W- comprises at most 60 carbon atoms.
In an embodiment W comprises at most 60 carbon atoms without counting any $-CH_2-CH_2O-$ radicals.
In an embodiment W comprises at most 50 carbon atoms.
In an embodiment W comprises at most 50 carbon atoms without counting any $-CH_2-CH_2O-$ radicals.
In an embodiment W comprises at most 40 carbon atoms.
In an embodiment W comprises at most 40 carbon atoms without counting any $-CH_2-CH_2O-$ radicals.
In an embodiment W comprises at most 30 carbon atoms.
In an embodiment W comprises at most 30 carbon atoms without counting any $-CH_2-CH_2O-$ radicals.
In an embodiment W comprises at most 20 carbon atoms.
In an embodiment W comprises at most 20 carbon atoms without counting any $-CH_2-CH_2O-$ radicals.
In an embodiment W comprises at most 10 carbon atoms.
In an embodiment W comprises at most 10 carbon atoms without counting any $-CH_2-CH_2O-$ radicals.
In an embodiment W comprises at most 10 oxygen atoms.
In an embodiment W comprises at most 10 oxygen atoms without counting any $-CH_2-CH_2O-$ radicals.
In an embodiment W comprises at most 5 oxygen atoms.
In an embodiment W comprises at most 5 oxygen atoms without counting any $-CH_2-CH_2O-$ radicals.
The crosslinked dextran hydrogel according to the invention is a dextran polymer wherein the central-linker $L(-)_i$ is a linear, or a branched polyethylene glycol (PEG) radical.
By branched PEG is meant various PEG arms connected by a linear, branched, or cyclic alkyl, or by an aromatic, comprising between 2 to 20 carbon atoms and may comprise heteroatoms such as nitrogen, oxygen, or sulphur.
In one embodiment, the crosslinked dextran hydrogel according to the invention is a dextran polymer wherein the central-linker $L(-)_i$ is a branched PEG radical which possesses at most 8 arms.
In an embodiment, the central-linker $L(-)_i$ is a PEG chosen among the PEG of formula I :

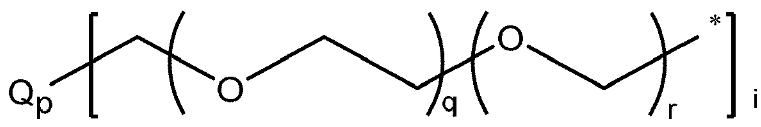
Formula I

Wherein:

- i is an integer comprised from 2 to 8 ($2 \leq i \leq 8$)
- p is an integer equal to 0 or 1, and if i=2 then p=0

- q is an integer comprised from 8 to 1000 ($8 \leq q \leq 1000$)
- r is an integer equal to 0 or 1
- Q is either a carbon atom, or a linear, branched, or cyclic alkyl chain, or an aromatic, comprising 2 to 10 carbon atoms and may comprise heteroatoms such as nitrogen, oxygen, or sulphur
- the * represents the sites of $f_4$, which is an amine function, or an ether, or a thioether function, or an amide function, or a carbamate function or a carbon-nitrogen covalent bond, or a carbon-aromatic carbon covalent bond, or a carbon-carbon covalent bond if the crosslinking process is made by a Native Chemical Ligation (NCL).

In an embodiment, the cross-linked dextran polymer according to the invention is a dextran polymer wherein $L(-)_i$ is a radical issued from a linear or branched mercaptopolyethyleneglycol comprising at least 2 sulfur atoms and comprising at most 8 arms, which

- number-average molecular weight (Mn) is comprised from 500 to 40 000 g/mol ($500 \leq Mn \leq 40\,000$ g/mol) or
- polymerisation degree (DP) is comprised from 8 to 1000 ($8 \leq DP \leq 1000$).

In an embodiment, the cross-linked dextran polymer according to the invention is a dextran polymer wherein $L(-)_i$ is a radical issued from a linear or branched mercaptopolyethyleneglycol comprising at least 2 sulfur atoms and comprising at most 8 arms, which

- Mn is comprised from 1000 to 25 000 g/mol ($1000 \leq Mn \leq 25\,000$ g/mol) or
- polymerisation degree (DP) is comprised from 15 to 600 ($15 \leq DP \leq 600$).

In an embodiment, the cross-linked dextran polymer according to the invention is a dextran polymer wherein $L(-)_i$ is a radical according to formula I, issued from the thiol polyethylene glycols or mercaptopoly(oxyethylenes) cited in the following table:

| Chemical Name | i | Mn (kg/mol) |
|---|---|---|
| Poly(ethylene glycol) dithiol | 2 | 10 |
| Poly(ethylene glycol) dithiol | 2 | 3.4 |
| Poly(ethylene glycol) dithiol | 2 | 1 |
| Pentaerythritol tetra(mercaptoethyl) polyoxyethylene | 4 | 5.2 |
| Pentaeryth ritol tetra(mercaptoethyl) polyoxyethylene | 4 | 20 |
| tripentaerythritol octa(mercaptoethyl) polyoxyethylene | 8 | 20 |

In an embodiment, the cross-linked dextran polymer according to the invention is a dextran polymer wherein $L(-)_i$ is a radical according to formula I issued from a pentaerythritol tetra(mercaptoethyl) polyoxyethylene, CAS# 188492-68-4.

In an embodiment, the cross-linked dextran polymer according to the invention is a dextran polymer wherein $L(-)_i$ is a radical according to formula I issued from a linear (mercaptoethyl)polyoxyethylene, CAS# 68865-60-1.

In an embodiment, the cross-linked dextran polymer according to the invention is a dextran polymer wherein $L(-)_i$ is a radical according to formula I, issued from a pentaerythritol poly(azide) polyoxyethylene cited in the following table:

| Chemical Name | i | Mn (kg/mol) |
|---|---|---|
| 2-arm PEG azide (Polyoxyethylene bis(azide)) | 2 | 5 |
| 2-arm PEG azide | 2 | 10 |
| 4-arm PEG azide, pentaerythritol core | 4 | 20 |
| 4-arm PEG azide, pentaerythritol core | 4 | 40 |

In an embodiment, the cross-linked dextran polymer according to the invention is a dextran polymer wherein $L(-)_i$ is a radical according to formula I, issued from a pentaerythritol poly(dibenzocyclooctyne) polyoxyethylene cited in the following table:

| Chemical Name | i | Mn (kg/mol) |
|---|---|---|
| 2-arm PEG DBCO Dibenzocycloctyne-PEG-Dibenzocycclotyne 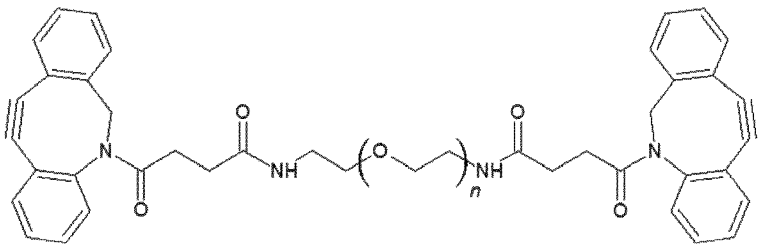 | 2 | 5 |
| 2-arm PEG DBCO | 2 | 10 |
| 4-arm PEG DBCO, pentaerythritol core 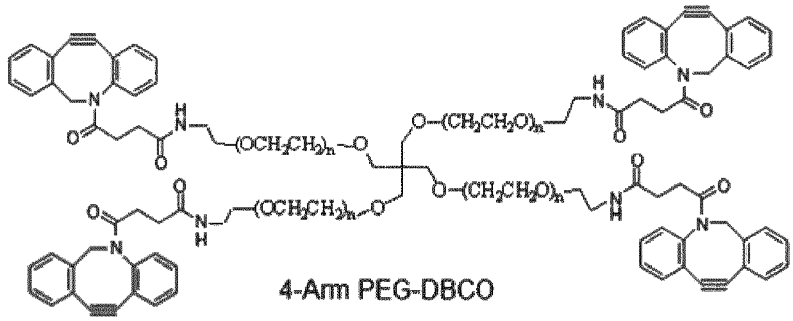  | 4 | 20 |
| 4-arm PEG DBCO, pentaerythritol core | 4 | 40 |

The hydroxyl functions of the dextran polymer Dx can be functionalised by at least one specific anionic group such as: alkyl carboxylate, sulphate anions, or sulfonate anions, or phosphate anions, or phosphonate anions.

In one embodiment, the hydroxyl functions of the dextran polymer Dx can be functionalised by sulphate anions in salified form, and optionally by alkyl carboxylate derivatives in salified form.

In another embodiment, the hydroxyl functions of the dextran polymer Dx can be functionalised by sulfonate anions in salified form, and optionally by alkyl carboxylate derivatives in salified form.

In another embodiment, the hydroxyl functions of the dextran polymer Dx -, can be functionalised by phosphate anions in salified form, and optionally by alkyl carboxylate derivatives in salified form.

In another embodiment, the hydroxyl functions of the dextran polymer Dx can be functionalised by phosphonate anions in salified form, and optionally by alkyl carboxylate derivatives in salified form.

In another embodiment, the hydroxyl functions of the dextran polymer Dx can be functionalised by alkyl carboxylate derivatives in salified form.

The specific anionic groups defined previously are chosen among the groups of formula II:

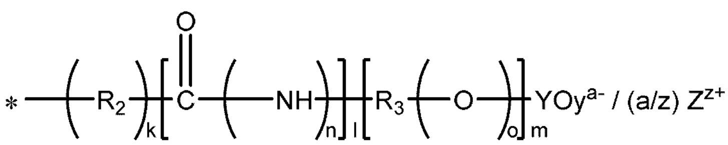

Formula II

Wherein:

- * represents the link to the O atoms of the dextran to form an ether function.
- y=2 or 3.
- When y=2, alkyl carboxylate derivatives, then:
    - ◦ Y=C and a=1.
    - ◦ k=1, l=0 and m=0.
    - ◦ $R_2$=Alkyl.

- When y=3, anionic group, then:
    - Y=S and a=1, or Y=P and a=2.
    - k=0 or 1.
    - I=0 or 1.
    - m=0 or 1.
    - n=1 or 2, in particular n=1.
    - o=0 or 1.
    - if I=1 then m=1.
    - $R_3$=linear, branched, or cyclic alkyl which may contain one heteroatom such as nitrogen, or aromatic, or PEG.
    - R2=Alkyl.
- And, Z is a counter ion, which can be an alkali metal and z=1, or which can be an alkaline earth metal and z=2.

In a preferred embodiment, the dextran backbone, Dx-, can be functionalised by sulphate anions in salified form, and optionally by alkyl carboxylate derivatives in salified form.

In another preferred embodiment, the dextran backbone, Dx-, can be functionalised by alkyl sulfonate anions in salified form, and optionally by alkyl carboxylate derivatives in salified form.

In another preferred embodiment, the dextran backbone, Dx-, can be functionalised by sulfonate anions in salified form, supported by an alkyl chain comprising a dimethyl-ammonium cation, and optionally by alkyl carboxylate derivatives in salified form.

In another preferred embodiment, the dextran backbone, Dx-, can be functionalised by alkyl carboxylate derivatives in salified form.

In an embodiment, the cross-linked dextran polymer bearing anionic groups according to the invention is a dextran polymer wherein the dextran polymer backbone is according to formula III,

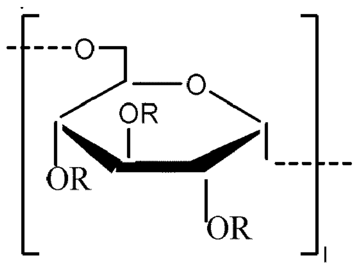
Formula III

wherein R is chosen among

- -H, a anionic group of formula II, or a -W- radical bearing a $L(-)_i$ crosslinker,
- i is comprised from 20 to 5000 ($20 \leq i \leq 5000$),
- -W- and $L(-)_i$ radicals having the previously defined meanings.

In an embodiment, the cross-linked dextran polymer according to the invention is a dextran polymer wherein the dextran polymer backbone is a dextran having a weight average molecular weight (Mw) comprised from 5 to 1000 kDa before cross-linking and substitution.

In other words, the cross-linked dextran polymer according to the invention is obtained after substitution and crosslinking of a native dextran polymer having a weight average molecular weight (Mw) comprised from 5 to 1000 kDa.

In an embodiment, the cross-linked dextran polymer according to the invention is a dextran polymer wherein the dextran polymer backbone is a dextran having a weight average molecular weight (Mw) comprised from 5 to 250 kDa before cross-linking and substitution.

In an embodiment, the cross-linked dextran polymer according to the invention is a dextran polymer wherein the dextran polymer backbone is a dextran having a weight average molecular weight (Mw) comprised from 5 to 100 kDa before cross-linking and substitution.

In an embodiment, the cross-linked dextran polymer according to the invention is a dextran polymer wherein the dextran polymer backbone is a dextran having a weight average molecular weight (Mw) comprised from 5 to 50 kDa before cross-linking and substitution.

In an embodiment, the cross-linked dextran polymer according to the invention is a dextran polymer wherein the dextran polymer backbone is a dextran having a weight average molecular weight (Mw) comprised from 5 to 25 kDa

before cross-linking and substitution.

In an embodiment, the cross-linked dextran polymer according to the invention is a dextran polymer wherein the dextran polymer backbone is a dextran having a weight average molecular weight (Mw) comprised from 250 to 1000 kDa before cross-linking and substitution.

In an embodiment, the cross-linked dextran polymer according to the invention is a dextran polymer wherein the dextran polymer backbone is a dextran having a weight average molecular weight (Mw) comprised from 10 to 500 kDa before cross-linking and substitution.

In an embodiment, the cross-linked dextran polymer according to the invention is a dextran polymer wherein the dextran polymer backbone is a dextran having a weight average molecular weight (Mw) comprised from 20 to 500 kDa before cross-linking and substitution.

In an embodiment, the cross-linked dextran polymer according to the invention is a dextran polymer wherein the dextran polymer backbone is a dextran having a weight average molecular weight (Mw) comprised from 20 to 100 kDa before cross-linking and substitution.

In an embodiment, the cross-linked dextran polymer according to the invention is a dextran polymer wherein the dextran polymer backbone is a dextran having a weight average molecular weight (Mw) comprised from 20 to 50 kDa before cross-linking and substitution.

In an embodiment, the cross-linked dextran polymer according to the invention is a dextran polymer wherein the dextran polymer backbone is a dextran having a weight average molecular weight (Mw) comprised from 40 to 250 kDa before cross-linking and substitution.

In an embodiment, the cross-linked dextran polymer according to the invention is a dextran polymer wherein the dextran polymer backbone is a dextran having a weight average molecular weight (Mw) comprised from 40 to 100 kDa before cross-linking and substitution.

In an embodiment, the cross-linked dextran polymer according to the invention is a cross-linked dextran polymer wherein the degree of substitution ($DS_1$) of the dextran backbone with the a -W- radical bearing a $L(-)_i$ crosslinker is comprised in the range from 0.001 to 0.4 ($0.001 \leq DS_1 \leq 0.4$).

In an embodiment, the cross-linked dextran polymer according to the invention is a cross-linked dextran polymer wherein the degree of substitution ($DS_1$) of the dextran backbone with the -W- radical bearing a $L(-)_i$ crosslinker is comprised in the range from 0.01 to 0.4 ($0.01 \leq DS_1 \leq 0.4$).

In an embodiment, the cross-linked dextran polymer according to the invention is a cross-linked dextran polymer wherein the degree of substitution ($DS_1$) of the dextran backbone with the -W- radical bearing a $L(-)_i$ crosslinker is comprised in the range from 0.05 to 0.4 ($0.05 \leq DS_1 \leq 0.4$).

In an embodiment, the cross-linked dextran polymer according to the invention is a cross-linked dextran polymer wherein the degree of substitution ($DS_1$) of the dextran backbone with the -W- radical bearing a $L(-)_i$ crosslinker is comprised in the range from 0.1 to 0.4 ($0.1 \leq DS_1 \leq 0.4$).

In an embodiment, the cross-linked dextran polymer according to the invention is a cross-linked dextran polymer wherein the dextran polymer backbone is a dextran having a weight average molecular weight (Mw) is from 5 to 250 kDa before cross-linking and substitution and the degree of substitution ($DS_1$) of the dextran backbone with the -W- radical bearing a $L(-)_i$ crosslinker groups is comprised in the range from 0.1 to 0.4 ($0.1 \leq DS_1 \leq 0.4$).

In an embodiment, the cross-linked dextran polymer according to the invention is a cross-linked dextran polymer wherein the dextran polymer backbone is a dextran having a weight average molecular weight (Mw) is from 20 to 100 kDa before cross-linking and substitution and the degree of substitution ($DS_1$) of the dextran backbone with the -W- radical bearing a $L(-)_i$ crosslinker groups is comprised in the range from 0.2 to 0.4 ($0.2 \leq DS_1 \leq 0.4$).

In an embodiment, the cross-linked dextran polymer according to the invention is a cross-linked dextran polymer wherein the dextran polymer backbone is a dextran having a weight average molecular weight (Mw) from 20 to 100 kDa before cross-linking and substitution and the degree of substitution ($DS_1$) of the dextran backbone with the -W- radical bearing a $L(-)_i$ crosslinker is comprised in the range from 0.2 to 0.3 ($0.2 \leq DS_1 \leq 0.3$).

In an embodiment, the cross-linked dextran polymer according to the invention is a cross-linked dextran polymer wherein the dextran polymer backbone is a dextran having a weight average molecular weight (Mw) is from 250 to 1000 kDa before cross-linking and substitution and the degree of substitution ($DS_1$) of the dextran backbone with the -W- radical bearing a $L(-)_i$ crosslinker is comprised in the range from 0.001 to 0.4 ($0.001 \leq DS_1 \leq 0.4$).

In an embodiment, the cross-linked dextran polymer according to the invention is a cross-linked dextran polymer wherein the dextran polymer backbone is a dextran having a weight average molecular weight (Mw) from 250 to 1000 kDa before cross-linking and substitution and the degree of substitution ($DS_1$) of the dextran backbone with the -W- radical bearing a $L(-)_i$ crosslinker is comprised in the range from 0.01 to 0.4 ($0.01 \leq DS_1 \leq 0.4$).

In an embodiment, the cross-linked dextran polymer according to the invention is a cross-linked dextran polymer wherein the dextran polymer backbone is a dextran having a weight average molecular weight (Mw) from 250 to 1000 kDa before cross-linking and substitution and the degree of substitution ($DS_1$) of the dextran backbone with the -W- radical bearing a $L(-)_i$ crosslinker is comprised in the range from 0.05 to 0.4 ($0.05 \leq DS_1 \leq 0.4$).

In an embodiment, the cross-linked dextran polymer according to the invention is a cross-linked dextran polymer wherein the dextran polymer backbone is a dextran having a weight average molecular weight (Mw) from 250 to 1000 kDa before cross-linking and substitution and the degree of substitution ($DS_1$) of the dextran backbone with the -W- radical bearing a $L(-)_i$ crosslinker groups is comprised in the range from 0.1 to 0.4 ($0.1 \leq DS_1 \leq 0.4$).

In an embodiment, the cross-linked dextran polymer according to the invention is a cross-linked dextran polymer wherein the degree of substitution ($DS_4$) of the dextran backbone with a radical of formula II is comprised in the range from 0.5 to 3 ($0.5 \leq DS_4 \leq 3$).

In an embodiment, the cross-linked dextran polymer according to the invention is a cross-linked dextran polymer wherein the degree of substitution ($DS_4$) of the dextran backbone with a radical of formula II is comprised in the range from 1 to 2.75 ($1 \leq DS_4 \leq 2.75$).

In an embodiment, the cross-linked dextran polymer according to the invention is a cross-linked dextran polymer wherein the degree of substitution ($DS_4$) of the dextran backbone with a radical of formula II is comprised in the range from 1.5 to 2.5 ($1.5 \leq DS_4 \leq 2.5$).

In an embodiment, the cross-linked dextran polymer according to the invention is a cross-linked dextran polymer wherein the degree of substitution ($DS_4$) of the dextran backbone with a radical of formula II is comprised in the range from 1.75 to 2.25 ($1.75 \leq DS_4 \leq 2.25$).

In an embodiment, the cross-linked dextran polymer according to the invention is a cross-linked dextran polymer wherein the degree of substitution of carboxylate ($DS_c$) of the dextran backbone is comprised in the range from 0.2 to 3 ($0.2 \leq DSc \leq 3$).

In an embodiment, the cross-linked dextran polymer according to the invention is a cross-linked dextran polymer wherein the degree of substitution of carboxylate ($DS_c$) of the dextran backbone is comprised in the range from 0.3 to 2.5 ($0.3 \leq DSc \leq 2.5$).

In an embodiment, the cross-linked dextran polymer according to the invention is a cross-linked dextran polymer wherein the degree of substitution of sulfate, sulfonate, phosphate, phosphonate ($DS_3$) of the dextran backbone is comprised in the range from 0.2 to 2.5 ($0.2 \leq DS_3 \leq 2.5$).

In an embodiment, the cross-linked dextran polymer according to the invention is a cross-linked dextran polymer wherein the degree of substitution of sulfate, sulfonate, phosphate, phosphonate ($DS_3$) of the dextran backbone is comprised in the range from 0.3 to 2.0 ($0.3 \leq DS_3 \leq 2.0$).

In an embodiment, the cross-linked dextran polymer according to the invention is a cross-linked dextran polymer having a molar ratio (DC) between the molar concentration of the -W- radical and the molar concentration of the reactive functions of the cross-linker $L(-)_i$ comprised in a range from 0.5 to 1.5 ($0.5 \leq DC \leq 1.5$).

In an embodiment, the cross-linked dextran polymer according to the invention is a cross-linked dextran polymer having a molar ratio between the molar concentration of the -W- radical and the molar concentration of the reactive functions of the cross-linker $L(-)_i$ is comprised in a range from 0.8 to 1.2 ($0.8 \leq DC \leq 1.2$).

In an embodiment, the cross-linked dextran polymer according to the invention is a cross-linked dextran polymer having a molar ratio between the molar concentration of the -W- radical and the molar concentration of the reactive functions of the cross-linker $L(-)_i$ is comprised in a range from 0.9 to 1.1 ($0.9 \leq DC \leq 1.1$).

In an embodiment, the cross-linked dextran polymer according to the invention is a cross-linked dextran polymer having a molar ratio between the molar concentration of the -W- radical and the molar concentration of the reactive functions of the cross-linker $L(-)_i$ is comprised in a range from 0.95 to 1.05 ($0.95 \leq DC \leq 1.05$).

In an embodiment, the cross-linked dextran polymer according to the invention is a cross-linked dextran polymer having a molar ratio between the molar concentration of the -W- radical and the molar concentration of the reactive functions of the cross-linker $L(-)_i$ is 1 (DC = 1).

In an embodiment, the cross-linked dextran polymer according to the invention is a cross-linked dextran polymer obtained from a reaction between the reactive function of the W precursor and the reactive function of the (L)I precursor where reactive function are present in the same concentration (DC = 1) and are comprised in a range going from 5 to 25 mM.

In an embodiment they are comprised in the range of 5 to 10 mM.

In an embodiment they are comprised in the range of 10 to 15 mM.

In an embodiment they are comprised in the range of 15 to 20 mM.

In an embodiment they are comprised in the range of 20 to 25 mM.

In an embodiment -W- is chosen among the radicals of formula IV.

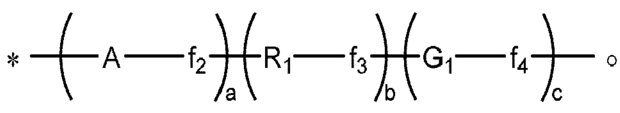

IV

Wherein

- * represents the site of $f_1$ and ° represents the site of attachment with L.
- a is an integer equal to 0 or 1.
- b is an integer equal to 0 or 1.
- c is an integer equal to 0 or 1.
- In one embodiment a=0, $f_1$ is an ether function, or a carbamate function.
- In one embodiment a=1,

  ◦ the divalent radical -A- is a linear, $-(CH_2)_{n1}-$ with $n_1$ an integer comprised from 1 to 7 ($1 \leq n_1 \leq 7$), branched, or cyclic alkyl derivative. It may also be branched by at least one hydroxyl group, $-CH_2-CH(OH)-(CH_2)_{n2}-$ with $n_2$ an integer comprised from 1 to 5 ($1 \leq n_2 \leq 5$); $f_1$ is an ether function, or a carbamate function, and $f_2$ is an amide function.
  Or,
  ◦ the divalent radical -A- is a linear polyether (PEG) derivative; $f_1$ is an ether function, or a carbamate function, and $f_2$ is an amide function. Or,
  ◦ in another embodiment the divalent radical -A- is a 4-Alkyl-1,4-triazole derivative or a 4-PEG-1,4-triazole derivative; $f_1$ is an ether function, or a carbamate function, and $f_2$ is a carbon-nitrogen covalent bond.
  Or,
  ◦ in another embodiment the divalent radical -A- is a 1-Alkyl-1,4-triazole derivative or a 1-PEG-1,4-triazole derivative; $f_1$ is an ether function, or a carbamate function, and $f_2$ is a carbon-aromatic carbon covalent bond.

- The divalent radical $-R_1-$ is a linear, branched, or cyclic alkyl derivative, and/or an aromatic derivative, and/or a polyether (PEG) derivative, which can contain heteroatoms such as nitrogen, oxygen, or sulphur.

  ◦ If b=0, then $f_1$ is an ether function, or a carbamate function.
  ◦ If b=1, then $f_1$ is an ether function, or a carbamate function, and $f_3$ is an amide function, or an amine function, or an ether function, or a thioether function, or a carbamate function, or a carbon-nitrogen covalent bond, or a carbon-aromatic carbon covalent bond or a carbon-carbon covalent bond if the crosslinking process is made by a Native Chemical Ligation (NCL).

- The divalent radical $-G_1-$ is a linear, branched, or cyclic alkyl derivative, or an aromatic derivative, which can contain heteroatoms such as: at most 5 nitrogen atoms, at most 10 oxygen atoms, at most 5 sulphur atoms, or at most one phosphorus atom. In a preferred embodiment, $-G_1-$ is a succinimide derivative, or an alkyl sulfone derivative which can contain one heteroatom such as oxygen or sulphur, or an ethyl amide derivative, or a 1,4-triazole derivative, or a multicycle derivative from a Diels-Alder reaction, or an aromatic phosphine derivative created by a Staudinger ligation, or a cysteine derivative coming from a Native Chemical Ligation.

  ◦ If c=0, then $f_1$, is an ether function, or a carbamate function.
  ◦ If c=1, then $f_1$, is an ether function, or a carbamate function, and $f_4$ is an amine function, or an amide function, or a carbamate function, or a thioether function, or an ether function, or a carbon-nitrogen covalent bond, or carbon-aromatic carbon covalent bond, or a carbon-carbon covalent bond if the crosslinking process is made by a Native Chemical Ligation (NCL).

In this embodiment, the cross-linked dextran polymer according to the invention is chosen among the dextran polymers of formula V.

$$\left[Dx - f_1 \left( A - f_2 \right)_a \left( R_1 - f_3 \right)_b \left( G_1 - f_4 \right)_c \right]_i L$$

Formula V

Wherein

- $f_1$, $f_2$, $f_3$, $f_4$, -A-, $-R_1-$, $-G_1-$ are defined as above in Formula IV, and
- Dx- is a dextran moiety, which can be substituted by specific anionic groups in salified form, and optionally by alkyl carboxylate derivatives in salified form as previously defined.
- The integer i is the valence of the central-linker L, and the number of, identical or different, [Dx-$f_1$-(A-

$f_2)_a$-$(R_1$-$f_3)_b$-$(G_1$-$f_4)_c$] radicals connected to L.

- The central-linker L is a polyether (PEG) derivative, which can be linear or branched.

In one embodiment L is linked to the same [Dx-$f_1$-(A-$f_2)_a$-$(R_1$-$f_3)_b$-$(G_1$-$f_4)_c$] radicals.
In one embodiment L is linked to different [Dx-$f_1$-(A-$f_2)_a$-$(R_1$-$f_3)_b$-$(G_1$-$f_4)_c$] radicals.
If a=0, then:

- In one embodiment, $f_1$ is an ether function.
- In another embodiment, $f_1$ is a carbamate function.

If a=1, then:

- In one embodiment, $f_1$ is an ether function.
- In another embodiment, $f_1$ is a carbamate function.

If a=0 and b=0, then:

- In one embodiment, $f_1$ is an ether function.
- In another embodiment, $f_1$ is a carbamate function.

If a=0 and b=1, then:

- In one embodiment, $f_1$ is an ether function.
- In another embodiment, $f_1$ is a carbamate function.

If a=b=c=0, then:

- In one embodiment, $f_1$ is an ether function.
- In another embodiment, $f_1$ is a carbamate function.

In one embodiment, the divalent radical -A- is a linear polyether (PEG) derivative chosen among the PEG of following formula:

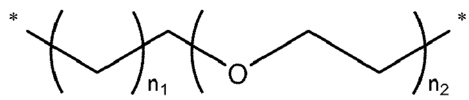

Wherein:

- $n_1$ is an integer equal to 0 or 1.
- $n_2$ is an integer comprised from 1 to 7 ($1 \leq n_1 \leq 7$).
- The * represent the sites of $f_1$ and $f_2$.
- In a preferred embodiment, the * represent the sites of $f_1$ and $f_2$, which are respectively ether and amide functions.

In another embodiment the divalent radical -A- is a 1-Alkyl-1,4-triazole derivative, or a 1-PEG-1,4-triazole derivative, chosen among the triazole derivative of following formula:

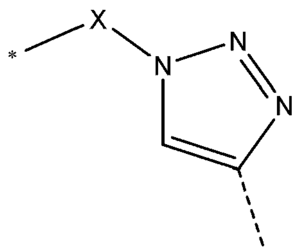

Wherein:

- X is either a linear *-$(CH_2)_{n1}$-* with $n_1$ an integer comprised from 1 to 7 ($1 \leq n_1 \leq 7$), branched, or cyclic alkyl derivative, or X is a PEG derivative.
- The * represents the site of $f_1$ and the dotted bond represents $f_2$.

In another embodiment the divalent radical -A- is a 4-Alkyl-1,4-triazole derivative, or a 4-PEG-1,4-triazole derivative, chosen among the triazole derivative of following formula:

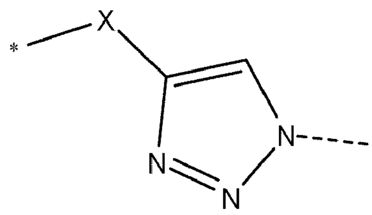

Wherein:

- X is either a linear *-$(CH_2)_{n1}$-* with $n_1$ an integer comprised from 1 to 7 ($1 \leq n_1 \leq 7$), branched, or cyclic alkyl derivative, or X is a PEG derivative.
- The * represents the site of $f_1$ and the dotted bond represents $f_2$.

If a=1, then:

- In one embodiment, $f_2$ is an amide function.
- In another embodiment, $f_2$ is a carbon-nitrogen covalent bond.
- In another embodiment, $f_2$ is a carbon-aromatic carbon covalent bond.

In one embodiment, the divalent radical -$R_1$- is a linear alkyl derivative, according to the following formula:

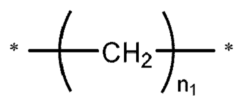

Wherein:

- $n_1$ is an integer comprised from 1 to 7 ($1 \leq n_1 \leq 7$).
- In one embodiment, if a=1, then the * represent the sites of $f_2$ and $f_3$.
- In another embodiment, if a=0, the * represent the sites of $f_1$ and $f_3$.

In another embodiment, the divalent radical -$R_1$- is a polyether (PEG) derivative, according to the following formula:

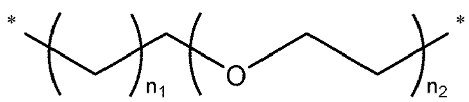

Wherein:

- $n_1$ is an integer equal to 0 or 1.
- $n_2$ is an integer comprised from 1 to 7 ($1 \leq n_2 \leq 7$).
- In one embodiment, if a=1, then the * represent the sites of $f_2$ and $f_3$.
- In another embodiment, if a=0, the * represent the sites of $f_1$ and $f_3$.

In another embodiment, if a=0, then the divalent radical -$R_1$- may be a branched alkyl, wherein at least one hydroxyl group is attached to the alkyl chain in β position from $f_1$, which is an ether function.

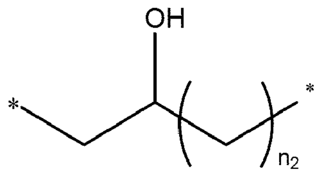

Wherein:

- $n_2$ is an integer comprised from 1 to 5 ($1 \leq n_2 \leq 5$).
- In another embodiment if a=0, the * represent the sites of $f_1$ and $f_3$.

In a preferred embodiment, the divalent radical $-R_1-$ is a linear alkyl derivative, according to the following formula:

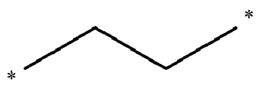

Wherein:
the * represent the sites of $f_2$, which is an amide function, and $f_3$, which is an amide function.
In a preferred embodiment, the divalent radical $-R_1-$ is a PEG derivative, according to the following formula:

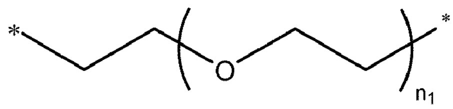

Wherein:

- $n_1$ is an integer comprised from 1 to 7 ($1 \leq n_1 \leq 7$).
- The * represent the sites of $f_2$ and $f_3$, which are two amide functions.

If b=1, then:

- In one embodiment, $f_3$ is an amine function.
- In another embodiment, $f_3$ is an ether function.
- In another embodiment, $f_3$ is a thioether function.
- In another embodiment, $f_3$ is an amide function.
- In another embodiment, $f_3$ is a carbamate function.
- In another embodiment, $f_3$ is a carbon-nitrogen covalent bond.
- In another embodiment, $f_3$ is a carbon-aromatic carbon covalent bond.
- In another embodiment, if the crosslinking process is made by a Native Chemical Ligation (NCL), then $f_3$ is a carbon-carbon covalent bond.

The nature of radical -G1- depends of the crosslinking process, below are described the different crosslinking process together with the G1 radicals.
In one embodiment, the crosslinking process is realized with a Michael addition with maleimide derivatives, or vinyl sulfone derivatives, or acrylamide derivatives.
In one embodiment, the divalent radical $-G_1-$ is a succinimide derivative according to the following formula:

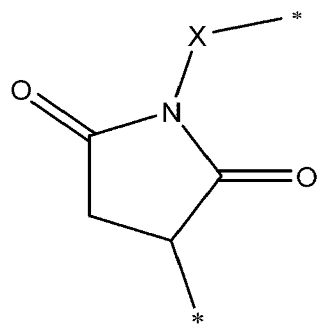

Wherein:

- X is either a linear *-$(CH_2)_{n1}$-* with $n_1$ an integer comprised from 1 to 7 ($1 \leq n_1 \leq 7$), branched, or cyclic alkyl derivative, or X is a PEG derivative.
- In one embodiment if b=1, then the * represent the sites of $f_3$ and $f_4$.
- In another embodiment if a=b=0, then the * represent the sites of $f_1$ and $f_4$.
- In another embodiment if a=1 and b=0, then the * represent the sites of $f_2$ and f4.
- In a preferred embodiment, X is an ethyl group and the * represent the sites of $f_2$, which is an amide function, and $f_4$, which is an thioether function.

In another embodiment, the divalent radical -$G_1$- is a diethyl sulfone derivative according to the following formula:

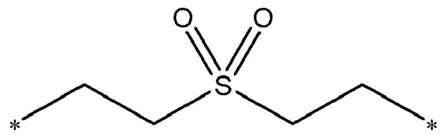

Wherein:

- In one embodiment if b=1, the * represent the sites of $f_3$ and $f_4$.
- In another embodiment if a=b=0, the * represent the sites of $f_1$ and $f_4$.
- In another embodiment if b=0, the * represent the sites of $f_2$ and $f_4$.
- In a preferred embodiment, the * represent the sites of $f_3$ and $f_4$, which are thioether functions.

In another embodiment, the divalent radical -$G_1$- is a sulfone derivative according to the following formula:

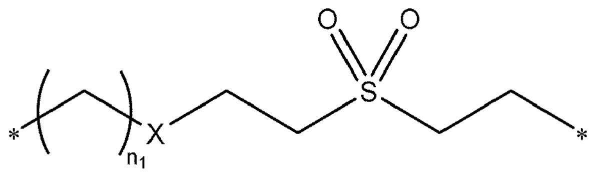

Wherein:

- $n_1$ is an integer comprised from 0 to 7 ($0 \leq n_1 \leq 7$).
- X is either an oxygen atom, or a sulphur atom, or a $CH_2$ group.
- In one embodiment if b=1, the * represent the sites of $f_3$ and $f_4$.
- In another embodiment if a=b=0, the * represent the sites of $f_1$ and $f_4$.
- In another embodiment if a=1 and b=0, the * represent the sites of $f_2$ and $f_4$.
- In preferred embodiment a=1, b=0, X is a sulphur atom, $n_1$=2, $f_2$ is an amide function, and $f_4$ is a thioether function.

In another embodiment, the divalent radical -$G_1$- is an acrylamide derivative according to the following formula:

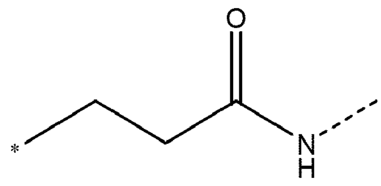

Wherein:

- In one embodiment, the * represents the site of $f_3$, which is an amine function, or an ether function, or a thioether function, and the dotted bond represents $f_4$, which is a carbon-nitrogen covalent bond.
- In another embodiment, the dotted bond represents f3, which is a carbon-nitrogen covalent bond, and the * represents the site of $f_4$, which is an amine function, or an ether function, or a thioether function.

In one embodiment, the crosslinking process is realized with a 1,3-cycloaddition between alkyne and azide derivatives, known as 1,3-dipolar cycloaddition or Huisgen reaction.

In one embodiment, the divalent radical $-G_1-$ is a 1,4-triazole derivative according to the following formula:

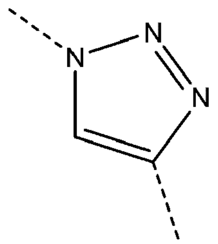

Wherein the two dotted bonds represent $f_3$ and $f_4$ which are covalent bonds or chemical functions defined previously and after.

In one embodiment, the crosslinking process is realized with a 1,3-cycloaddition between strain alkyne and azide derivatives, known as Strain-promoted azide-alkyne cycloaddition, or SPAAC.

In one embodiment, the divalent radical $-G_1-$ is a triazole derivative according to the following formula:

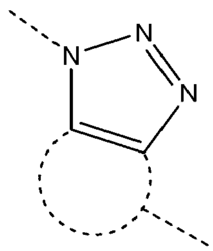

Wherein:

- The dotted circle represents a cyclooctene derivative, coming from strained cyclooctyne, which can contain one heteroatom such as nitrogen, oxygen, or sulphur, and is optionally functionalised by linear, branched, or cyclic alkyl derivatives comprising between 2 to 20 carbon atoms, or by aromatics derivatives, or by heteroatoms such as nitrogen, oxygen, or sulphur, or halogens, especially fluorine.
- The two dotted bonds represent $f_3$ and $f_4$, which are covalent bonds or chemical functions defined previously and after.
- In another embodiment if a=b=0, the two dotted bonds represent $f_1$ and $f_4$.
- In another embodiment if a=1 and b=0, the two dotted bonds represent $f_2$ and f4.
- In a preferred embodiment, the dotted bonds represent $f_2$, which is an amide function, and $f_4$, which is a carbon-nitrogen covalent bond.

In another embodiment, the divalent radical $-G_1-$ is a triazole derivative according to the following formula:

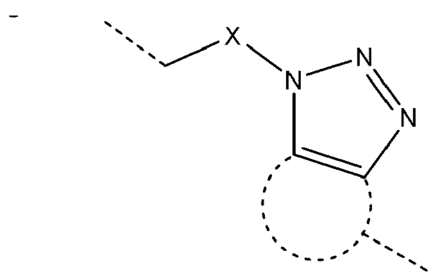

Wherein:

- The dotted circle represents a cyclooctene derivative, coming from strained cyclooctyne, which can contain one heteroatom such as nitrogen, oxygen, or sulphur, and is optionally functionalised by linear, branched, or cyclic alkyl derivatives comprising between 2 to 20 carbon atoms, or by aromatics derivatives, or by heteroatoms such as nitrogen, oxygen, or sulphur, or halogens, especially fluorine.
- X is either a linear $*\text{-}(CH_2)_{n1}\text{-}*$ with $n_1$ an integer comprised from 1 to 7 ($1 \leq n_1 \leq 7$), branched, or cyclic alkyl derivative, or X is a PEG derivative.
- The two dotted bonds represent $f_3$ and $f_4$, which are covalent bonds or chemical functions defined previously and after.
- In another embodiment if a=b=0, the * represent the sites of $f_1$, and the dotted bond represents $f_4$.
- In another embodiment if a=1 and b=0, the * represent the sites of $f_1$, and the dotted bond represents $f_4$.
- In a preferred embodiment, X is a PEG derivative, the dotted bonds represent $f_2$ and $f_4$, which are amide functions

In a preferred embodiment, the divalent radical $-G_1-$ is a triazole derivative according to the following formula:

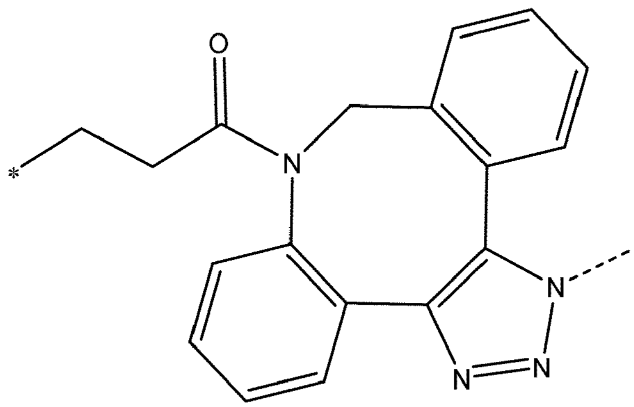

Or

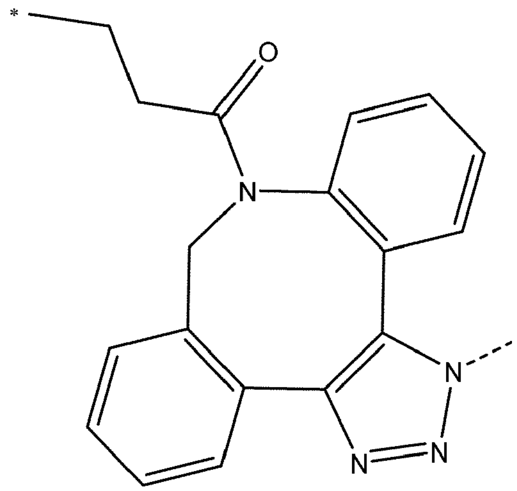

Wherein: the * represents the site of $f_2$, which is an amide function, and the dotted bond represents $f_4$, which is a carbon-nitrogen bond.

In another preferred embodiment, the divalent radical $-G_1-$ is a triazole derivative according to the following formula:

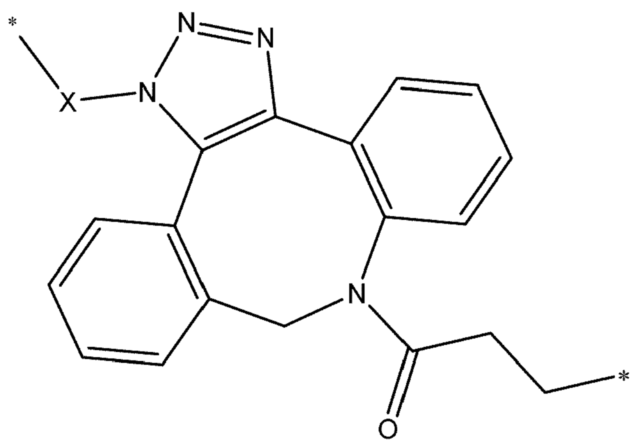

Or

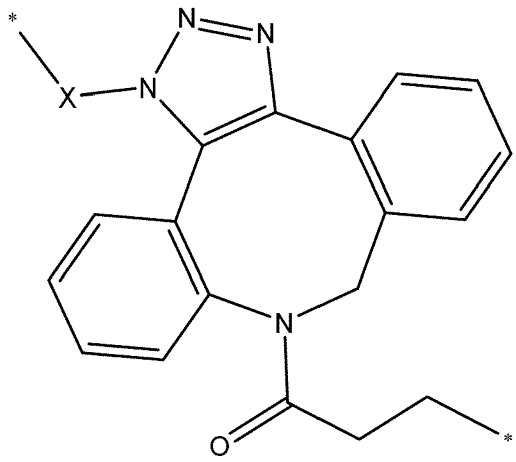

Wherein:

- X is a PEG derivative.
- The * represent the sites of $f_2$ and $f_4$, which are amide functions.

In another embodiment, the divalent radical $-G_1-$ is a triazole derivative according to the following formula:

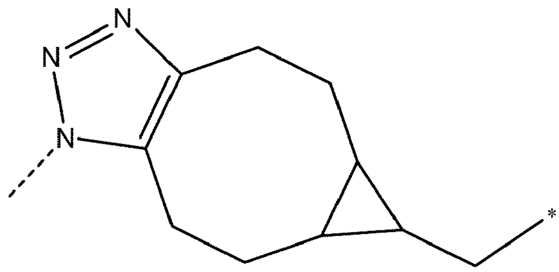

Wherein: the * represents the site of $f_3$, and the dotted bond represents $f_4$.

In another embodiment, the divalent radical $-G_1-$ is a triazole derivative according to the following formula:

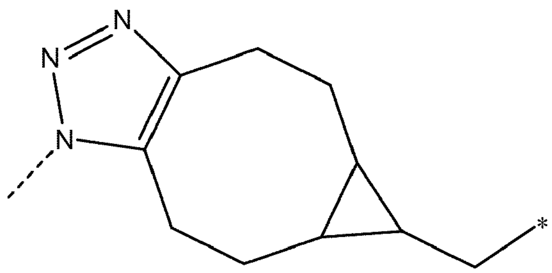

Wherein, the dotted bond represents $f_3$, and the * represents the site of $f_4$.

In one embodiment, the crosslinking process is realized with a Diels-Alder cycloaddition between maleimide and furane derivatives.

In one embodiment, the divalent radical $-G_1-$ is a multiple cycle derivative, composed of one succinimide moiety, according to the following formula:

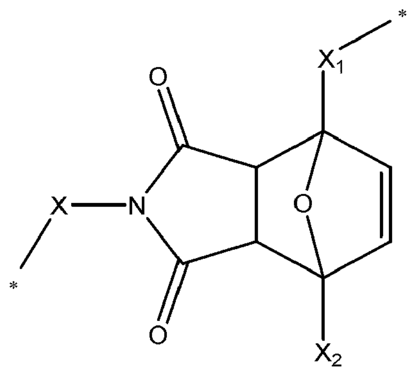

Wherein:

- X is either a linear *-$(CH_2)_{n1}$-* with $n_1$ an integer comprised from 1 to 7 ($1 \leq n_1 \leq 7$), branched, or cyclic alkyl derivative, or X is a PEG derivative.
- $X_1$ is either a linear *-$(CH_2)_{n1}$-* with $n_1$ an integer comprised from 0 to 7 ($0 \leq n_1 \leq 7$), branched, or cyclic alkyl derivative.
- $X_2$ is either -H or -Me.
- In one embodiment if a=b=1, the * represent the sites of $f_3$ and $f_4$.
- In another embodiment if a=b=0, the * represent the sites of $f_1$ and $f_4$.
- In another embodiment if a=1 and b=0, the * represent the sites of $f_2$ and $f_4$.

In one embodiment, the crosslinking process is realised with an inverse electron-demand Diels-Alder reaction or IEDDA, between tetrazine and norbornene derivatives.

In one embodiment, the divalent radical -$G_1$- is a multiple cycle derivative, composed of one pyridazine moiety, according to the following formula:

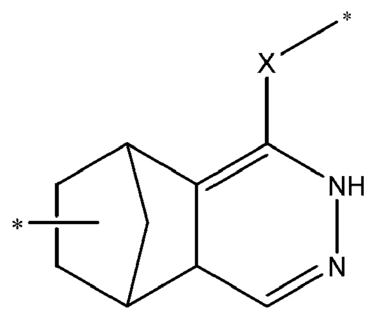

Wherein:

- X is either a linear *-$(CH_2)_{n1}$-* with $n_1$ an integer comprised from 1 to 7 ($1 \leq n_1 \leq 7$), branched, or cyclic alkyl derivative, or X is an aromatic derivative, or X is a PEG derivative.
- In one embodiment if a=b=1, the * represent the sites of $f_3$ and $f_4$.
- In another embodiment if a=b=0, the * represent the sites of $f_1$ and $f_4$.
- In another embodiment if a=1 and b=0, the * represent the sites of $f_2$ and $f_4$.

In another embodiment, the divalent radical -$G_1$- is a multiple cycle derivative, composed of one pyridazine moiety, according to the following formula:

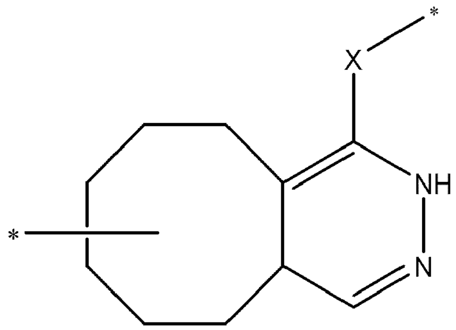

Wherein:

- X is either a linear *-$(CH_2)_{n1}$-* with $n_1$ an integer comprised from 1 to 7 ($1 \leq n_1 \leq 7$), branched, or cyclic alkyl derivative, or X is an aromatic derivative, or X is a PEG derivative.
- In one embodiment if a=b=1, the * represent the sites of $f_3$ and $f_4$.
- In another embodiment if a=b=0, the * represent the sites of $f_1$ and $f_4$.
- In another embodiment if a=1 and b=0, the * represent the sites of $f_2$ and $f_4$.

In one embodiment, the crosslinking process is realised with a Staudinger ligation, between an aromatic phosphine and an azide derivative.

In one embodiment, the divalent radical -$G_1$- is an aromatic derivative, according to the following formula:

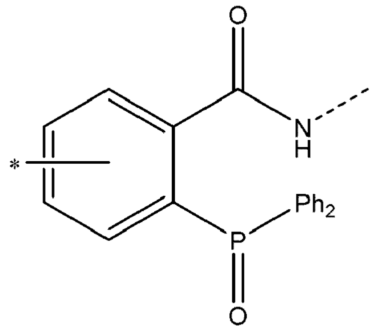

Wherein:

- In one embodiment if a=b=1, the * represent the sites of $f_3$ and $f_4$.
- In another embodiment if a=b=0, the * represent the sites of $f_1$ and $f_4$.
- In another embodiment if a= 1 and b=0, the * represent the sites of $f_2$ and $f_4$.

In one embodiment, the crosslinking process is realized with a Native Chemical Ligation (NCL), between thioester and N-terminal cysteine derivatives.

In one embodiment, the divalent radical -$G_1$- can be formalised according to the following formula:

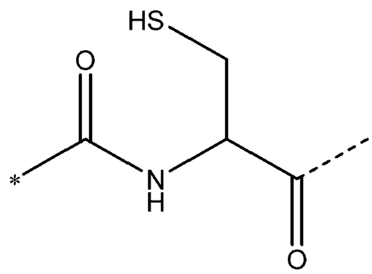

Wherein:

- The dotted line represents a carbon-nitrogen covalent bond.
- In one embodiment if a=b=1, the * and the dotted line represent the sites of $f_3$ and $f_4$.
- In another embodiment if a=b=0, the * and the dotted line represent the sites of $f_1$ and $f_4$.
- In another embodiment if a=1 and b=0, the * and the dotted line represent the sites of $f_2$ and $f_4$.

If c=1, then:

- In one embodiment, $f_4$ is an amine function.
- In another embodiment, $f_4$ is an ether function.
- In another embodiment, $f_4$ is a thioether function.
- In another embodiment, $f_4$ is an amide function.
- In another embodiment, $f_4$ is a carbamate function
- In another embodiment, $f_4$ is a carbon-nitrogen covalent bond.
- In another embodiment, $f_4$ is a carbon-aromatic carbon covalent bond.
- In another embodiment, if the crosslinking process is made by a Native Chemical Ligation (NCL), then $f_4$ is a carbon-carbon covalent bond.

The invention also concerns the dextran polymers of formula VIII before the crosslinking reaction.

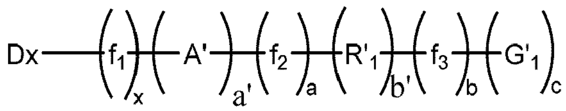
Formula VIII

Wherein

- $f_1$, $f_2$, $f_3$, Dx are defined as above if none of a, a', b and b' are equal to 0,
- and
- x equal 0 or 1.
- if a, a', b and b' are equal to 0, x is equal to 0 and Dx is a dextran polymer backbone according to formula III, wherein R is chosen among -H or a anionic group of formula II,
- if one of b' and c is not equal to 0 -A'- is -A- as defined above,
- if b', b and c are equal to 0, a is equal to 0 and -A'- is the precursor of -A- before the crosslinking reaction.
- if c is not equal to 0, $-R'_1-$ is $-R_1-$ as defined above and $-G'_1-$ is the precursor of $-G_1-$.
- if c is equal to 0, b is equal to 0 and $-R'_1-$ is the precursor of $-R_1-$ before the crosslinking , reaction.

In an embodiment -A'- is an alkyl carboxylate derivative or a poly(oxyethylene)carboxylate derivative, or an alkyl azide derivative, or a poly(oxyethylene)azide derivative, or a propargyl derivative, or a poly(oxyethylene)propargyl derivative, or a 2-hydroxyalkyl carboxylate, or a 2-hydroxyalkylamine.

-A'- as carboxylate derivatives can be formalized with the following formulas:

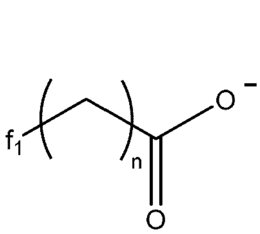
Or
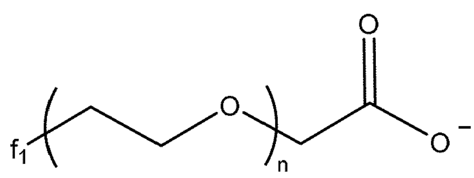

-A'- as azide derivatives can be formalized with the following formulas:

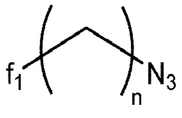
Or
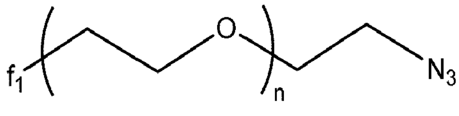

-A'- as propargyl derivatives can be formalized with the following formulas:

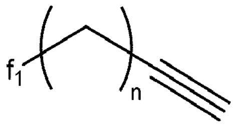

Or

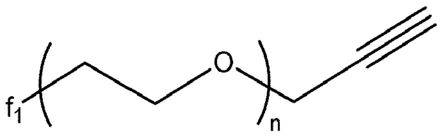

-A'- as a 2-hydroxyalkyl carboxylate derivative can be formalized with the following formula:

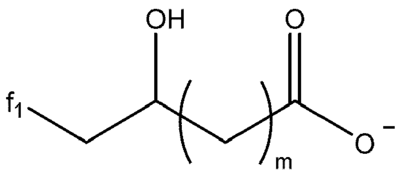

-A'- as a 2-hydroxyalkylamine derivatives can be formalized with the following formula:

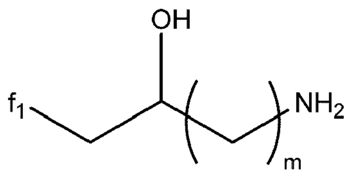

Wherein:

n is an integer comprised from 1 to 7 ($1 \leq n \leq 7$)
m is an integer comprised from 1 to 5 ($1 \leq m \leq 5$)
$f_1$ is defined as previously

In an embodiment, if a=1, -$R'_1$- is an alkyl radical or a poly(oxyethylene) radical bearing a terminal amine, or a terminal hydroxyl, or a terminal thiol, or a terminal carboxylate, or a terminal azide, or a terminal alkyne.

-$R'_1$- can be formalized as follows:

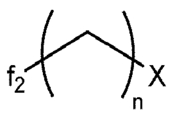

Or

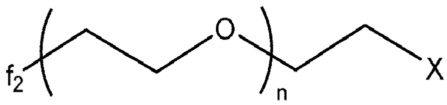

-$R'_1$- as carboxylate derivatives can be formalized with the following formulas:

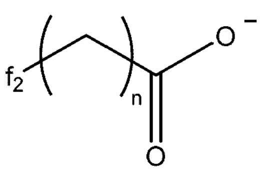

Or

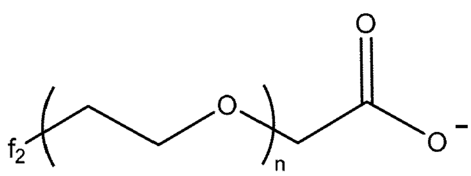

-$R'_1$- as azide derivatives can be formalized with the following formulas:

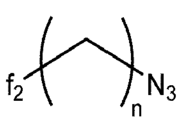

Or

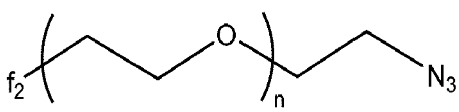

Wherein:

- n is an integer comprised from 1 to 7 ($1 \leq n \leq 7$)
- X= $-NH_2$, or -OH, or -SH
- $f_2$ is defined as previously

Or, in another embodiment, if a=0, then -R'1- is a branched alkyl, wherein at least one hydroxyl group is attached to the alkyl chain in β position from f1, and having a terminal hydroxyl, or a terminal thiol, or a terminal azide, or a terminal alkyne.

R'1 can be formalized as follows:

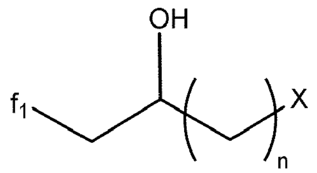

Wherein:

- n is an integer comprised from 1 to 5 ($1 \leq n \leq 5$)
- X= $-NH_2$, or -OH, or -SH, or -N3, or -C=CH
- $f_1$ is defined as previously

Or, in another R'$_1$ is a propargyl derivatives, and can be formalized with the following formulas:

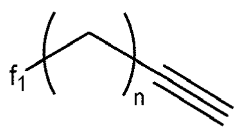

Or

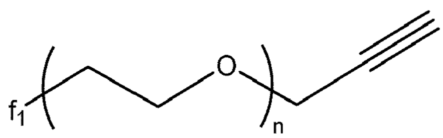

Wherein:

- n is an integer comprised from 1 to 7 ($1 \leq n \leq 7$)
- $f_1$ is defined as previously

In an embodiment G'1 is a maleimide derivative, or a vinylsulfone derivative, or a strained cyclooctyne derivative, or an azide derivative, or propargyl derivative, or a furane derivative, or an acrylamide derivative, or a norbornene derivative, or a trans-cyclooctene derivative, or a tetrazine derivative, or an aromatic phosphine, or a cysteine, or a thioester, or a thiol derivative, or an amine derivative, or a hydroxyl derivative.

G'1 as a thiol, an amine or a hydroxyl derivative can be formalized as follow:

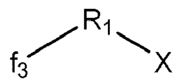

G'$_1$ as a maleimide can be formalized as follows:

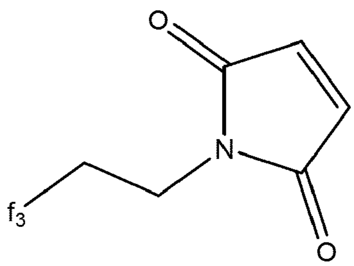

G'$_1$ as a vinylsulfone can be formalized as follows:

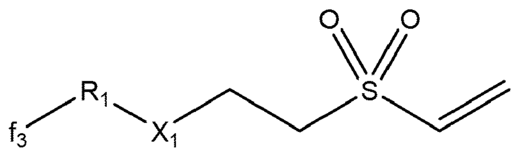

G'$_1$ as a strained cyclooctyne can be formalized as follows:

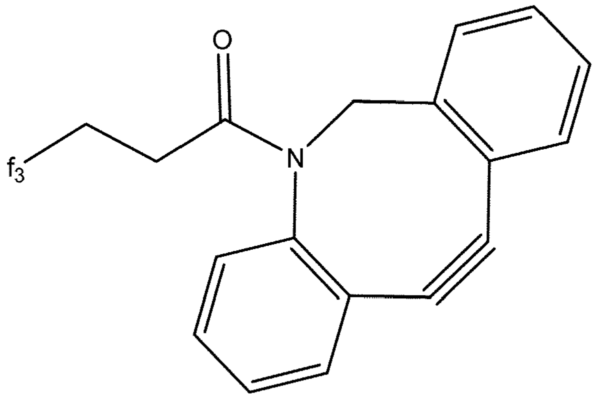

Or

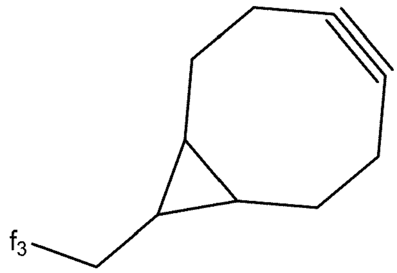

G'$_1$ as azide derivatives can be formalized with the following formula:

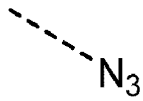

G'$_1$ as propargyl derivatives can be formalized with the following formula:

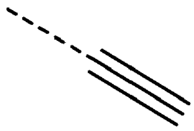

G'$_1$ as furane derivatives can be formalized with the following formula:

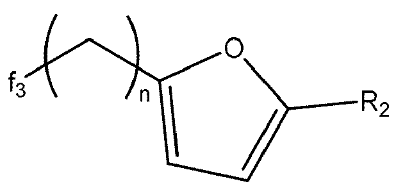

G'$_1$ as acrylamide derivatives can be formalized with the following formula:

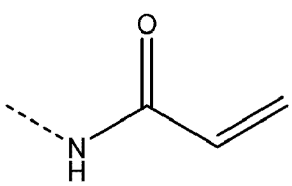

G'$_1$ as norbornene derivatives can be formalized with the following formula:

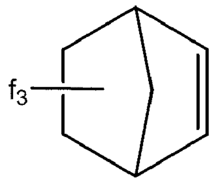

G'$_1$ as trans-cyclooctene derivatives can be formalized with the following formula:

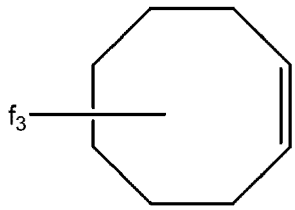

G'$_1$ as tetrazine derivatives can be formalized with the following formula:

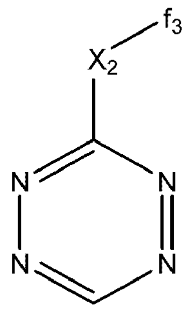

G'$_1$ as aromatic phosphine derivative can be formalized with the following formula:

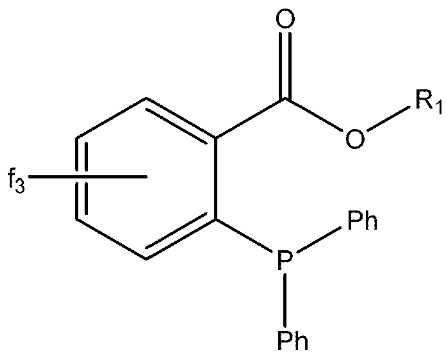

G'$_1$ as cysteine derivatives can be formalized with the following formula:

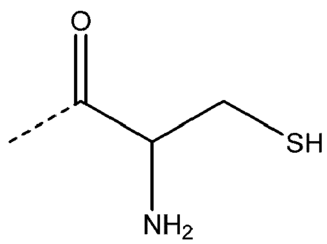

G'$_1$ as thioester derivatives can be formalized with the following formula:

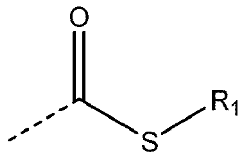

Wherein:

- X= $-NH_2$, or -OH, or -SH
- $X_1$= -O-, or -S-
- n is an integer equal to 0 or 1
- $R_1$= Alkyl
- $X_2$= -CH2-, or aromatic
- R2= -H, or -CH3
- $f_3$ is defined as previously

The dotted bonds represent $f_3$, which is a carbon-nitrogen covalent bond, or a carbon-carbon covalent bond.

The invention also concerns a hydrogel comprising the cross-linked dextran polymer according to the invention.

The invention also concerns a Kit comprising :

- A solution of dextran polymer of formula VIII before the crosslinking reaction:

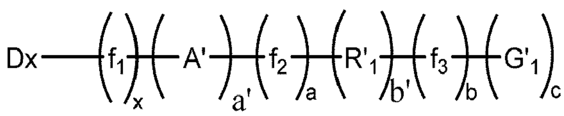

Formula VIII

Wherein

- $f_1$, $f_2$, $f_3$, $f_4$, Dx are defined as in formula IV if none of a, a', b and b' are not equal to 0,
- and
- if a, a', b and b' are equal to 0, x is equal to 0 and Dx is a dextran polymer backbone is according to formula III, wherein R is chosen among -H or a anionic group of formula II,
- if one of b' and c is not equal to 0 -A' is A as defined above,
- if b', b and c are equal to 0, a is equal to 0 and A' is the precursor of A before the crosslinking reaction.
- if c is not equal to 0, $R'_1$ is $R_1$ as defined above and $G'_1$ is the precursor of $G_1$.
- if c is equal to 0, b is equal to 0 and $R'_1$ is the precursor of $R_1$ before the crosslinking , reaction.

- a solution of a thiol polyethylene glycol, a mercaptopoly(oxyethylenes), a pentaerythritol poly(oxyethylene) azide or a pentaerythritol poly(dibenzocyclooctyne) polyoxyethylene.
- biological cells,
- a solution of non crosslinked sodium hyaluronate.

In an embodiment the hydrogel is transparent.

By "transparent" is meant that in conditions disclosed in Example C21 of application PCT/EP2022/050466 for visual inspection an observer considered the sample transparent compared to the standard 2 (6 NTU) and/or the UV absorbance of the hydrogel as measured in Example C21 of application PCT/EP2022/050466 is lower than 0.06 (Absorbance Units).

In an embodiment the hydrogel is visually transparent and has a UV absorbance < 0.06 (Abs. Units).

In an embodiment the hydrogel according to the invention is characterized in that Tan $\delta$ is lower than 1.

In the present specification, Tan $\delta$ is the ratio of the storage modulus (also called elastic modulus) G' to the loss modulus G" (Tan $\delta$ = G'/G").

In an embodiment the hydrogel according to the invention is characterized in that Tan $\delta$ is less than or equal to 0.5.

In an embodiment the hydrogel according to the invention is characterized in that Tan $\delta$ is less than or equal to 0.1.

In an embodiment the hydrogel according to the invention is characterized in that Tan $\delta$ is less than or equal to 0.05.

In an embodiment the hydrogel according to the invention is characterized in that Tan $\delta$ is less than or equal

to 0.01.

In an embodiment the hydrogel according to the invention is characterized in that after swelling in water the cross-linked dextran polymer concentration is comprised from 0.01 to 0.2 g/g.

In an embodiment the hydrogel according to the invention is characterized in that after swelling in water the cross-linked dextran polymer concentration is comprised from 0.03 to 0.1 g/g.

In an embodiment the hydrogel according to the invention is characterized in that after swelling in water the cross-linked dextran polymer concentration is comprised from 0.05 to 0.1 g/g.

In an embodiment, the hydrogel is translucid.

In another embodiment the hydrogel is transparent.

In an embodiment, the hydrogel has a Young modulus comprised between 1 to 200 kPa.

In an embodiment, the hydrogel has a Young modulus comprised between 5 to 200 kPa.

In an embodiment, the hydrogel has a Young modulus comprised between 20 to 200 kPa.

In an embodiment, the hydrogel has a Young modulus comprised between 30 to 200 kPa.

In an embodiment, the hydrogel has a Young modulus comprised between 50 to 200 kPa.

In an embodiment, the hydrogel has a Young modulus comprised between 30 to 180 kPa.

In an embodiment, the hydrogel has a Young modulus comprised between 50 to 150 kPa.

In an embodiment, the hydrogel has a Young modulus comprised between 5 to 100 kPa.

In an embodiment, the hydrogel has a Young modulus comprised between 10 to 90 kPa.

In an embodiment, the hydrogel has a Young modulus comprised between 10 to 75 kPa.

In an embodiment, the hydrogel has a G' comprised from 0.5 to 70 kPa.

In an embodiment the hydrogel has a compression deformation at break of more than or equal to 10 %.

In an embodiment the hydrogel has a compression deformation at break of more than or equal to 15 %.

In an embodiment the hydrogel has a compression deformation at break of more than or equal to 20 %.

In an embodiment the hydrogel has a compression deformation at break of more than or equal to 25 %.

In an embodiment the hydrogel has a compression deformation at break of more than or equal to 30 %.

In an embodiment the hydrogel has a compression deformation at break of more than or equal to 35 %.

In an embodiment the hydrogel has a compression deformation at break of more than or equal to 40 %.

In an embodiment the hydrogel has a compression deformation at break of more than or equal to 45 %.

In an embodiment the hydrogel has a compression deformation at break of more than or equal to 50 %.

In an embodiment the hydrogel has a compression deformation at break of more than or equal to 55 %.

In an embodiment the hydrogel has a compression deformation at break of more than or equal to 60 %.

In an embodiment the hydrogel has a traction deformation at break of more than or equal to 10 %.

In an embodiment the hydrogel has a traction deformation at break of more than or equal to 15 %.

In an embodiment the hydrogel has a traction deformation at break of more than or equal to 20 %.

In an embodiment the hydrogel has a traction deformation at break of more than or equal to 25 %.

In an embodiment the hydrogel has a traction deformation at break of more than or equal to 30 %.

In an embodiment the hydrogel has a traction deformation at break of more than or equal to 35 %.

In an embodiment the hydrogel has a traction deformation at break of more than or equal to 40 %.

In an embodiment the hydrogel has a swelling ratio of more than 0.7.

In an embodiment the hydrogel has a swelling ratio of more than 0.8.

In an embodiment the hydrogel has a swelling ratio of more than 0.9.

In an embodiment the hydrogel has a swelling ratio of more than 1.

In an embodiment the hydrogel has a swelling ratio of more than 1.1.

In an embodiment the hydrogel has a swelling ratio of more than or equal to 1.2.

In an embodiment the hydrogel has a swelling ratio of more than or equal to 1.3.

In an embodiment the hydrogel has a swelling ratio of more than or equal to 1.4.

In an embodiment the hydrogel has a swelling ratio of more than or equal to 1.5.

In an embodiment the hydrogel has a swelling ratio of more than or equal to 1.6.

In an embodiment the hydrogel has a swelling ratio of less than or equal to 5.

In an embodiment the hydrogel has a swelling ratio of less than or equal to 4.

In an embodiment the hydrogel has a swelling ratio of less than or equal to 3.

In an embodiment the hydrogel has a swelling ratio of less than or equal to 2.8.

In an embodiment the hydrogel has a swelling ratio of less than or equal to 2.5.

In an embodiment the hydrogel has a swelling ratio of less than or equal to 2.3.

In an embodiment the hydrogel has a water content of at least 80 wt%.

In an embodiment the hydrogel has a water content of at least 85 wt%.

In an embodiment the hydrogel has a water content of at least 90 wt%.

In an embodiment the hydrogel has a water content of at least 97 wt%.

In an embodiment the hydrogel has a water content of at least 96 wt%.
In an embodiment the hydrogel has a water content of at least 95 wt%.
In an embodiment the hydrogel has a water content of at least 94 wt%.
In an embodiment the hydrogel has a water content of at least 93 wt%.
In an embodiment the hydrogel has a water content of at most 99 wt%.
In an embodiment the hydrogel has a water content of at most 98 wt%.
In an embodiment the hydrogel according to the invention is characterized in that it further comprises biological cells.
In an embodiment the cells are cells from human or animal origin.
In an embodiment the cells are cell lines.
In an embodiment the cells are stem-cells derived.
In an embodiment the stem cells are chosen from embryonic-stem cells, from induced-pluripotent-stem-cells or from mesenchymal-stem-cells.
In an embodiment the cells are primary cells.
In an embodiment the cells are proteins, hormones or peptide secreting cells.
In an embodiment the cells are chosen from:

- insulin secreting cells for diabetes treatment
- Factor VIII or Factor IX secreting cells for hemophilia treatment and
- β-glucocerebrosidase secreting cells for Gaucher disease.

In an embodiment the hydrogel according to the invention is characterized in that insulin secreting cells are chosen into the group of pancreatic cells.
In an embodiment the hydrogel according to the invention is characterized in that insulin secreting cells are Langherans islets.
In an embodiment the hydrogel according to the invention is characterized in that the biological cells are pseudoislets.
The invention also concerns the use of a cross-linked dextran copolymer according to the invention into the form of a hydrogel to prepare a cells composition.
In an embodiment the cells are chosen amongst one or multiple type of cells, either isolated or aggregated, which may secrete active principles.
In an embodiment, the cross-linked dextran polymer according to the invention is a dextran polymer bearing anionic groups of formula II wherein the at least divalent radical $L(-)_i$ is covalently bound to the dextran polymer backbone with i -W- - radicals, wherein i is 2, 4 or 8.
In an embodiment, the cross-linked dextran polymer according to the invention is a dextran polymer bearing anionic groups of formula II wherein the at least divalent radical $L(-)_i$ is covalently bound to the dextran polymer backbone with i -W- - radicals, wherein, i is 2.
In an embodiment, the cross-linked dextran polymer according to the invention is a dextran polymer bearing anionic groups of formula II wherein the at least divalent radical $L(-)_i$ is covalently bound to the dextran polymer backbone with i -W- - radicals, wherein, i is 4.
In an embodiment, the cross-linked dextran polymer according to the invention is a dextran polymer bearing anionic groups of formula II wherein the at least divalent radical $L(-)_i$ is covalently bound to the dextran polymer backbone with i -W- - radicals, wherein, i is 8.
The cross-linking step is a gelation step that leads to the formation of a hydrogel according to the invention.
The hydrogel formation kinetic is function of the temperature and could be modulated by the reactant's concentrations, pH and temperature.
In an embodiment the time to obtain a hydrogel according to the invention is comprised from 1 minute to 6 hours.
In an embodiment the cross-linking step is carried out for 1 hour.
In an embodiment the temperature of the cross-linking step is comprised from 4°C to room temperature (20-25°C) and could vary between the step of mixing and the step of gelation and moulding.
In an embodiment the mixing is performed at 4°C and the gelation is carried out at room temperature (20-25°C) for 1 hour.
In an embodiment the mixing is performed at room temperature (20-25°C).
In an embodiment the mixing is performed at 4°C or room temperature (20-25°C) and the gelation is carried out at room temperature (20-25°C) for 1 hour.
In an embodiment, the gelation is carried out at 37 °C.
In an embodiment, after cross-linking or gelation, the hydrogel is swelled in a buffer solution, the pH of the buffer solution is comprised from 5 to 8, preferably from 6 to 8 and more preferably from 6.8 to 7.5.

In an embodiment, the buffer solution is a PBS solution at pH 7.4.

In an embodiment, the buffer solution is a Tris solution at pH 7.4.

In an embodiment, the buffer solution is a Tris solution at pH 8.

In an embodiment the swelling allows the hydrogel mass being increased by 1, 2, 3 or 4 compared to its initial mass.

The invention also concerns a process to synthetize a cross-linked dextran polymer according to the invention, into the form of a hydrogel, comprising the steps of:

a) preparation of a sterile solution comprising a dextran bearing anionic groups of formula II and at least two precursors of -W-
b) preparation of a sterile solution of a precursor of $L(-)_i$
c) addition of the sterile solution obtained from step b) to the solution obtained from step a),
d) the addition being directly done in a mould or the solutions are introduced into a mould after being mixed,
e) cross-linking and gelation, for example at room temperature (20-25°C) or at 37°C,
f) unmoulding and swelling to obtain an hydrogel.

In an embodiment steps c) and d) are done simultaneously.

In an embodiment, the swelling is done into a PBS solution at pH 7,4.

Dextrans bearing anionic groups of formula II are prepared by grafting or substitution on the hydroxyl groups borne by the dextrans. In an embodiment the dextrans bearing anionic groups of formula II are prepared by grafting or substituting of the carboxymethyl groups borne by the carboxymethyl dextrans.

In an embodiment of the process according to the invention an active pharmaceutical ingredient (API) is entrapped into the hydrogel.

The invention also concerns a therapeutic use of the hydrogel according to the invention as a therapeutic implant to administer the API to a mammal.

The invention also concerns a process to prepare a hydrogel comprising biological cells comprising the steps of:

a) preparation of a sterile solution comprising a dextran bearing anionic groups of formula II and at least two precursors of -W-
b) preparation of a sterile solution of a precursor of $L(-)_i$ chosen among a thiol polyethylene glycol, a mercaptopoly(oxyethylenes), a pentaerythritol poly(oxyethylene) azide or a pentaerythritol poly(dibenzocyclooctyne) polyoxyethylene
c) preparation of a sterile solution of hyaluronate de sodium,
d) preparation of a sterile suspension of biological cells,
e) mixing the sodium hyaluronate solution obtained from step c) with precursor solution from the step b)
f) mixing the biological cells suspension obtained from step d) and the solution obtained from the step e) or from step a)
g) mixing the solution obtained from step f) and the solutions obtained from step e),
h) addition of the sterile solution obtained from step a) or e) which is not used in step g) to the solution obtained from step f),
i) the addition of step g) being either done directly in a mould or the solutions are introduced into a mould after being mixed,
j) cross-linking and gelation reaction at room temperature (20-25°C),
k) unmoulding and swelling to obtain an hydrogel comprising biological cells.

In an embodiment, the mould is a Ring Net.

In an embodiment, cross-linking and gelation reaction are performed at room temperature (20-25°C),

In an embodiment, cross-linking and gelation reaction are performed at a controlled temperature comprise between 15°C and 37°C.

In an embodiment, the swelling is done into a PBS solution at pH 7.4.

In an embodiment the hydrogel according to the invention is characterized in that it further comprises biological cells.

In an embodiment the cells are cells from human or animal origin.

In an embodiment the cells are cell lines.

In an embodiment the cells are stem-cells derived.

In an embodiment the stem cells are chosen from embryonic-stem cells, from induced-pluripotent-stem-cells or from mesenchymal-stem-cells.

In an embodiment the cells are primary cells.

In an embodiment the cells are protein(s), hormone(s) or peptide(s) secreting cells.

In an embodiment the cells are chosen from

- insulin secreting cells for diabetes treatment
- Factor VIII or Factor IX secreting cells for hemophilia treatment and
- β-glucocerebrosidase secreting cells for Gaucher disease.

In an embodiment the hydrogel according to the invention is characterized in that insulin secreting cells are chosen into the group of pancreatic cells.
In an embodiment the hydrogel according to the invention is characterized in that insulin secreting cells are Langherans islets.
In an embodiment the hydrogel according to the invention is characterized in that the biological cells are pseudoislets.
The invention also concerns a therapeutic use of the hydrogel according to the invention for treating a disorder or disease in a mammal wherein the disorder or disease is due to lack or malfunction of endocrine function of pancreas organ.
The invention also concerns a hydrogel for use as a medicament.
The invention also concerns a hydrogel for use in the treatment of a disease such as diabetes.
The invention also concerns an implantable device comprising at least a hydrogel according to the invention and obtained according to the process of the invention.
The invention also concerns an implant consisting of the hydrogel according to the invention.
The invention also concerns an implant comprising the hydrogel according to the invention.
The invention also concerns an implant comprising the hydrogel according to the invention and cells or islets.
In an embodiment, at least 50 % of the surface the hydrogel is directly in contact with the medium in which it is implanted.
In an embodiment, at least 75 % of the surface the hydrogel is directly in contact with the medium in which it is implanted.
In an embodiment, at least 90 % of the surface the hydrogel is directly in contact with the medium in which it is implanted.
In an embodiment, at least 95 % of the surface the hydrogel is directly in contact with the medium in which it is implanted.
In an embodiment, 99 % of the surface the hydrogel is directly in contact with the medium in which it is implanted.
In an embodiment, at least 50 % of the surface the hydrogel is directly in contact with the exterior of the device or implant.
By « directly in contact with the exterior » means there is no separation between the hydrogel and the exterior, for example no wall made of a non-hydrogel material between the hydrogel and the exterior of the device or implant.
In an embodiment, at least 75 % of the surface the hydrogel is directly in contact with the exterior of the device or implant.
In an embodiment, at least 90 % of the surface the hydrogel is directly in contact with the exterior of the device or implant.
In an embodiment, at least 95 % of the surface the hydrogel is directly in contact with the exterior of the device or implant.
In an embodiment, at least 99 % of the surface the hydrogel is directly in contact with the exterior of the device or implant.
In an embodiment, 100 % of the surface the hydrogel is directly in contact with the exterior of the device or implant.
The cells or the API are entrapped into the maze of cross-linked dextran hydrogel.
In this specification the word "entrapped" is equivalent to "encapsulated" or "encapsulation".
The hydrogel matrix allows passage of small molecules e.g. nutrients and API, API being entrapped into the hydrogel or secreted by the entrapped cells.
Typically, API are hormone and peptide drugs chosen amongst PTH protein, insulin and coagulation factors.
In an embodiment, the mesh size of the matrix is immunoisolant and stops the T lymphocytes in order to preserve the cells.
In an embodiment, this mesh size is less than 1 μm.
In another embodiment it is less that 100 nanometers, preferably less than 10 nanometers, and more preferably around 5 nanometers.
In an embodiment the invention concerns an implant comprising a ring, a net, the hydrogel according to the invention and cells.
The ring and net structure allow the hydrogel to be easily manipulated, a good resistance to manipulation, including for implantation. This is also true even with a hydrogel having a larger mesh size (for example with a lower DS

of W and lower concentrations of reactive groups during crosslinking).

Figure 1 represents an implant (1) comprising a hydrogel (11) comprising cells or islets (not represented), a ring (12) and a net (13). Upper part of the ring, lower part of the ring and net can be glued together (not represented).
Figure 2 represents an implant (1) comprising a hydrogel (11) comprising cells or islets (not represented), a ring (12) and a net (13), where the hydrogel is concave.
Figure 3 is a top view of an implant (1) comprising a hydrogel (11) comprising cells or islets (not represented), a ring (12) and a net (13).
Figure 4 represents an implant (1) comprising an hydrogel without cells (20) sandwiching an hydrogel comprising cells (21). The upper part of 20 being optional.

In an embodiment the ring has an internal diameter of 10 to 100 mm.
In an embodiment the ring has an internal diameter of 15 to 50 mm.
In an embodiment the ring has a diameter of 0.5 to 5 mm.
In an embodiment the ring has a diameter of 0.5 to 10 mm.
In an embodiment the ring has a diameter of 0.5 to 5 mm.
In an embodiment the ring has a total (lower plus upper part and glue) thickness of 100 to 3000 μm.
In an embodiment the ring has a total (lower plus upper part and glue) thickness of 150 to 2000 μm.
In an embodiment the ring has a total thickness of 200 to 5000 μm.
In an embodiment the ring has a total thickness of 500 to 3000 μm.
In an embodiment the ring has a rectangular, square or round section.
In an embodiment the ring material is a bioinert material.
In an embodiment, the ring material is a biocompatible elastomer.
In an embodiment the ring material is chosen from the group consisting of silicone, in particular PDMS, polyurethanes, polyether, polyether polyester copolymers and polypropylene oxide.
In an embodiment the ring material is silicone.
In an embodiment the ring material is PDMS.
In an embodiment the net is non-biodegradable.
In an embodiment the net is biocompatible.
In an embodiment the net is non absorbable.
In an embodiment the net is a surgical mesh.
In an embodiment the filament material of the net material is chosen among the group consisting of Polypropylene, Polyethylene, polyester, in particular PET, PTFE, PVDF (polyvinylidene fluoride) and ePVDF (extended PVDF).
In an embodiment the filament material of the net is chosen among the group consisting of Polypropylene, polyester, in particular PET, PTFE and PVDF (polyvinylidene fluoride).
In an embodiment the filament material of the net is chosen among the group consisting of Polypropylene and polyester, in particular PET.
In an embodiment the filament material of the net is chosen among the group consisting of Polypropylene.
In an embodiment the filament material of the net is chosen among the group consisting of is polyester, in particular PET.
In an embodiment the net has a thickness ranging from 50 to 500 μm.
In an embodiment the m net has a thickness ranging from 100 to 300 μm.
In an embodiment, the filament diameter is ranging from 0.08 to 0.2 mm.
In an embodiment the pore size is ranging from 0.4 to 4 mm.
In an embodiment the pore size is ranging from 0.6 to 2 mm.
In an embodiment fabric of the net is chosen from the group consisting of knitted fabric, warp knitted fabric, woven fabric, non-woven fabric.
In an embodiment fabric of the net is chosen from the group consisting of warp knitted fabric, in particular multifilament.
In an embodiment the net is treated in order to increase the hydrophilicity.
In an embodiment the net is treated with a base, in particular on polyester, more particularly on PET.
In an embodiment this treatment is functionalisation of the surface from reactive function, such as -OH, -COOH, and reactive molecules or polymer.
The grafted polymer could thus expose reactive functions for a further reaction with the hydrogel or a precursor of hydrogel, such as a thiol function.
In an embodiment this treatment is done by adsorption of synthetic polymers, such as poloxamers or polyvinyl pyrrolidone (PVP) or of natural polymers, such as collagen, or of surfactants after a chemical or physical treatment.
In an embodiment the net remains below the exterior end of the ring.

In an embodiment the net is not in contact with the exterior of the implant comprising a ring, a net and the hydrogel.
In an embodiment the glue is biocompatible.
In an embodiment the glue is a biocompatible silicone glue, such as Silbione MED ADH 4200 supplied by Elkem.
In an embodiment the glue remains below the exterior end of the ring.
For example, a warp knit Polyester surgical net fabric type PETKM3002 (1x0.9mm pore size) supplied by SurgicalNet™ was treated in NaOH 1M for 5 hours at 70°C and rinsed with deionized water and ethanol 96%. The treatment led to an increased hydrophilicity of the net fabric leading to an improved wetting with aqueous solutions.
For example, a ring net construct may be obtained following the process below:

- Biocompatible PDMS sheets supplied by Grace Biolabs or Interstate Speciality Product is cut in a form of a square incorporating a circular empty disc using a stainless steel punch,
- A part of the treated polyester surgical net described is introduced in between two square PDMS pieces: The two PDMS pieces and the surgical net are glued together with biocompatible silicone glue (Silbione MED ADH 4200 supplied by Elkem). The circular empty discs are aligned, and the surgical net is kept tense during gluing,
- then, the square construct is cut with a strainless steel punch to obtain the final object constituted by two PDMS rings sandwiching a surgical net and glued together, and
- The pieces are washed with a solution of poloxamer F127 at 1% and rinsed with water before steam sterilization.

In an embodiment, the implant can be obtained by the following process:

- Hydrogel compositions are incorporated in the Ring Net constructs. The concentrated polymer solutions are mixed with a pipette and a controlled volume of the mixture is introduced in a ring net construct adhering to a glass slide,
- Crosslinking leading to gelation is carried out. Then the Ring Net + Hydrogel composition is introduced in a Tris 150 mM /NaCl 30 mM / Cystein 10 mM solution at pH 8 or in PBS at pH 7.4,
- The hydrogel was rinsed with PBS solution without cysteine and further immersed in the PBS solution overnight at 37°C. The hydrogel piece was then stored in PBS solution at 4°C until being used.

Hydrogel/Ring Net implants can then be easily manipulated with tweezers and are foldable for the need of surgical implantation. Moreover, it is possible to fix the ring with sutures.
The hydrogel volume can be adjusted with the internal diameter and the thickness or the ring net construct. For the same ring net construct the hydrogel volume can be adjusted to control the convexity / concavity of the hydrogel above the ring level.
In an embodiment, the hydrogel comprises a first layer of the hydrogel wich does not comprise cells or islets and a second layer of the hydrogel which comprises cells or islets.
Such a structure may be obtained with a process as disclosed in this application, but with two steps of adding hydrogel precursors first step is adding hydrogel precursors without cells or islets as a first layer, and the second step is adding the hydrogel precursor with cells or islets while the gelation of the first step is not finished, in particular at a time corresponding to 5 to 25 % of the gelation time of the first hydrogel, as a second layer.

## Part A - CHEMISTRY

### Example A1: Synthesis of substituted dextrans

**Table 1:** List of synthesized polysaccharides

| **Polysaccharides** | **Structure** |
|---|---|
| Polysaccharide 1 | 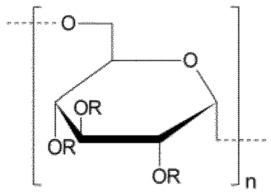<br>Mw (Dextran) = 40 kg/mol<br>n = 205<br>R = H or 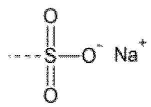 or 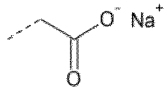 or 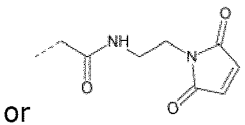<br>$DS_3$ = 1.5<br>$DS_2$ = 0.8<br>$DS_1$ = 0.25 |
| Polysaccharide 2 | 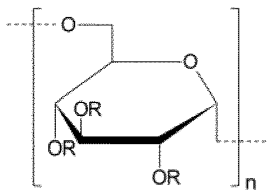<br>Mw (Dextran) = 40 kg/mol<br>n = 205<br>R = H or 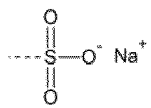 or 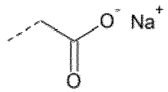 or 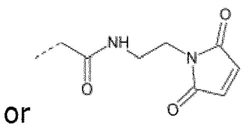<br>$DS_3$ = 1.9<br>$DS_2$ = 0.5<br>$DS_1$ = 0.23 |

(continued)

| Polysaccharides | Structure |
|---|---|
| Polysaccharide 3 | 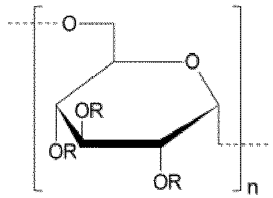<br>Mw (Dextran) = 40 kg/mol<br>n = 205<br>R = H or 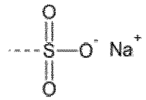 or 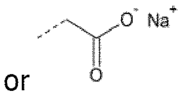 or 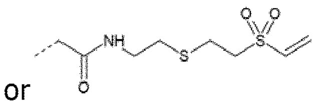<br>$DS_3$ = 1.5<br>$DS_2$ = 0.8<br>$DS_1$ = 0.28 |

(continued)

| Polysaccharides | Structure |
|---|---|
| Polysaccharide 4 | 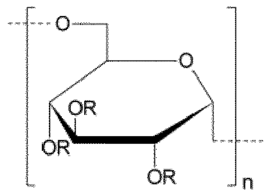<br>Mw (Dextran) = 40 kg/mol<br>n = 205<br><br>R = H or 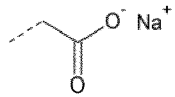 or 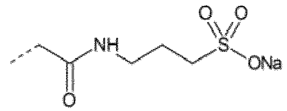<br>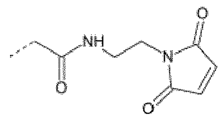<br>or<br>$DS_3$ = 1.15<br>$DS_2$ = 2.1<br>$DS_1$ = 0.25 |

(continued)

| Polysaccharides | Structure |
|---|---|
| Polysaccharide 5 | 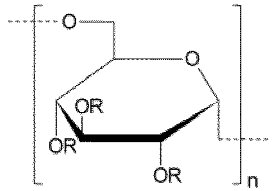<br>Mw (Dextran) = 40 kg/mol<br>n = 205<br>R = H or<br>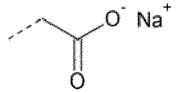 or 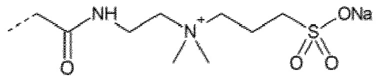<br>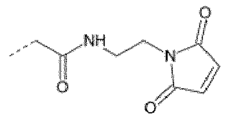<br>or<br>$DS_3$ = 0.7<br>$DS_2$ = 2.1<br>$DS_1$ = 0.22 |

(continued)

| Polysaccharides | Structure |
|---|---|
| Polysaccharide 6 | 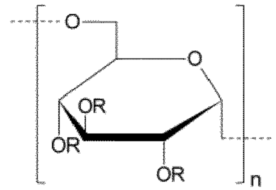<br>Mw (Dextran) = 40 kg/mol<br>n = 205<br>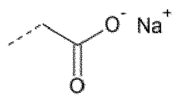 or 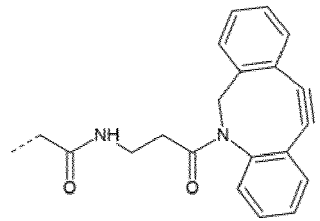<br>R = H or<br>$DS_2$ = 2.1<br>$DS_1$ = 0.28 |
| Polysaccharide 7 | 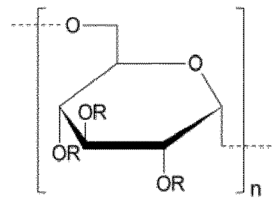<br>Mw (Dextran) = 40 kg/mol<br>n = 205<br>R = H or 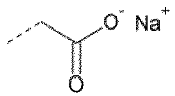 or 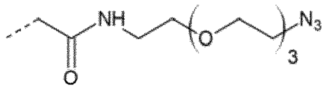<br>$DS_2$ = 2.1<br>$DS_1$ = 0.28 |

**Polysaccharide 1** - Dextran Methyl Carboxylate Sulfate and Maleimide

Polysaccharide 1.1 - Dextran Methyl Carboxylate

50 g (0.31 mol of glucoside units, 0.93 mol of hydroxyl functional groups) of dextran having a weight-average molar mass of 40 kg/mol (Pharmacosmos, degree of polymerization n = 205), are dissolved in water (225 g/L) at 30°C, then $NaBH_4$ (2 x 58 mg, 2 x 1.54 mmol) is added every 30 minutes and the mixture is stirred at 30°C for 1 h. To this solution is added sodium chloroacetate (72 g, 0.62 mol) and the mixture is heated at 65°C for 1 h. 10 N NaOH (103 mL, 1.03 mol) is then slowly added over 1.5 h and the mixture is stirred at 65°C for 1 h. The mixture is diluted with water (85 mL), cooled to room temperature, neutralized with acetic acid and then purified by ultrafiltration on PES membrane (MWCO 5 kDa) against phosphate buffer pH 7, then water. The polysaccharide 1.1 concentration of the final solution is determined by dry extract, and then an acid/base assay is carried out in order to determine the degree of substitution with methyl carboxylate.
According to the dry extract: [polysaccharide 1.1] = 46.3 mg/g
According to the acid/base assay, degree of substitution with methylcarboxylate ($DS_2$) = 0.8

Polysaccharide 1.2 - Dextran Methyl Carboxylic acid

550 g of the solution of polysaccharide 1.1 obtained above (46.3 mg/g, $DS_2$ = 0.8, 25.5 g, 112.6 mmol of glucoside units) is protonated with an acidified sulfonic resin (Purolite C100H, 2.0 eq/L, 200 mL, 400 mmol) for 2 h. The resulting solution is filtered, then freeze-dried.

Polysaccharide 1.3 - Dextran Methyl Carboxylate Sulfate

10.0 g (48.0 mmol of glucoside units) of freeze-dried polysaccharide 1.2 are dissolved in a mixture of DMF (320 mL) and formamide (80 mL). After complete dissolution, 2-methyl-2-butene (80 mL, 755.2 mmol) is slowly added. A $SO_3 \cdot$DMF complex (58.8 g, 383.9 mmol) is rapidly added, and the reaction mixture is stirred at 30°C for 3 h. The mixture is neutralized by slow addition of 5 % aq. $NaHCO_3$ (800 mL) and purified by ultrafiltration on PES membrane (MWCO 5 kDa) against 30 % v:v EtOH in NaCl (9 g/L in water), NaCl (9 g/L in water), then water. The polysaccharide 1.3 concentration of the final solution is determined by dry extract, and the degree of substitution with sulfate is determined by liquid chromatography after complete sulfate hydrolysis of a representative sample.
According to the dry extract: [polysaccharide 1.3] = 39.6 mg/g
According to the LC analysis, degree of substitution with sulfate ($DS_3$)= 1.5

Polysaccharide 1 - Dextran Methyl Carboxylate Sulfate and Maleimide

To 303 g of the solution of polysaccharide 1.3 obtained above (39.6 mg/g, DS3 = 1.5, DS2 = 0.8, 12.0 g, 31.64 mmol of glucoside units), 2-hydroxypyridine 1-oxide (HOPO) (1.76 g, 15.82 mmol) is added and the mixture is cooled to 4°C. To this solution are added N-(2-aminoethyl)maleimide hydrochloride (Mal) (1.68 g, 9.49 mmol) and N-ethyl-N'-(3-dimethylaminopropyl)carbodiimide hydrochloride (EDC) (3.03 g, 15.82 mmol) and the reaction mixture is stirred at 4°C for 2 h. Two additional additions of EDC (3.03 g, 15.82 mmol) are performed every 2 h. The mixture is diluted with phosphate buffer pH 7, then purified by ultrafiltration on PES membrane (MWCO 5 kDa) against phosphate buffer pH 7, NaCl (9 g/L in water) and then water. The polysaccharide 1 concentration of the final solution is determined by dry extract, and the degree of substitution with maleimide is determined by 1H NMR in D2O. The final solution is stored at -20°C.
According to the dry extract: [polysaccharide 1] = 22.2 mg/g
According to 1H NMR (D2O), degree of substitution with maleimide (DS1)=0.25

**Polysaccharide 2** - Dextran Methyl Carboxylate Sulfate and Maleimide

Polysaccharide 2.1 - Dextran Methyl Carboxylate

Using a process similar to the one used for the preparation of polysaccharide 1.1, starting from a dextran having a weight-average molar mass of 40 kg/mol (0.25 mol of glucoside units, 0.75 mol of hydroxyl functional groups, degree of polymerization n = 205), and with sodium chloroacetate (35.9 g, 0.31 mol) and 10 N NaOH (82 mL, 0.82 mol) at 60°C, polysaccharide 2.1 is obtained.
According to the dry extract: [polysaccharide 2.1] = 44.2 mg/g
According to the acid/base assay, degree of substitution with methylcarboxylate ($DS_2$) = 0.5

Polysaccharide 2.2 - Dextran Methyl Carboxylic acid

Using a process similar to the one used for the preparation of polysaccharide 1.2, starting from polysaccharide 2.1 (44.2 mg/g, DS2 = 0.5, 12.0 g, 59.4 mmol of glucoside units), polysaccharide 2.2 is obtained.

Polysaccharide 2.3 - Dextran Methyl Carboxylate Sulfate

Using a process similar to the one used for the preparation of polysaccharide 1.3, starting from polysaccharide 2.2 (4.0 g, 20.9 mmol of glucoside units), polysaccharide 2.3 is obtained.

According to the dry extract: [polysaccharide 2.3] = 22.0 mg/g

According to the LC analysis, degree of substitution with sulfate ($DS_3$)= 1.9

Polysaccharide 2 - Dextran Methyl Carboxylate Sulfate and Maleimide

Using a process similar to the one used for the preparation of polysaccharide 1, starting from polysaccharide 2.3 (22.0 mg/g, $DS_2$ = 0.5, $DS_3$ = 1.9, 4.6 g, 11.6 mmol of glucoside units), and with N-(2-aminoethyl) maleimide hydrochloride (615 mg, 3.48 mmol), polysaccharide 2 is obtained.

According to the dry extract: [polysaccharide 2] = 12.3 mg/g

According to $^1H$ NMR ($D_2O$), degree of substitution with maleimide ($DS_1$)=0.23

**Polysaccharide 3** - Dextran Methyl Carboxylate Sulfate and Vinyl Sulfone

To 280 g of solution of a polysaccharide 1.3 (35.7 mg/g, $DS_3$ = 1.5, $DS_2$ = 0.8, 10.0 g, 26.37 mmol of glucoside units), HOPO (1.46 g, 13.18 mmol) is added and the mixture is cooled to 4°C. To this solution are added 2-[[2-(ethenylsulfonyl)ethyl]thio]ethanamine hydrochloride (VS) (1.83 g, 7.91 mmol) (synthesized according: S. A. Stewart et al., Soft Matter, 2018, 14, 8317), $Et_3N$ (1.10 mL, 7.91 mmol) and EDC (2.53 g, 13.18 mmol) and the reaction mixture is stirred between 4°C and 25°C for 2 h. Two additional additions of EDC (2.53 g, 13.18 mmol) are performed every 2 h. The mixture is diluted with NaCl (9 g/L in water), then purified by ultrafiltration on PES membrane (MWCO 5 kDa) against NaCl (9 g/L in water), carbonate buffer pH 10, NaCl (9 g/L in water), phosphate buffer pH 7, NaCl (9 g/L in water), and then water. The polysaccharide 3 concentration of the final solution is determined by dry extract, and the degree of substitution with vinyl sulfone is determined by $^1H$ NMR in $D_2O$. The final solution is stored at -20°C.

According to the dry extract: [polysaccharide 3] = 21.8 mg/g

According to $^1H$ NMR ($D_2O$), degree of substitution with vinyl sulfone ($DS_1$)=0.28

**Polysaccharide 4** - Dextran Methyl Carboxylate Sulfonate and Maleimide

Polysaccharide 4.1 - Dextran Methyl Carboxylate

65 g (0.4 mol of glucoside units, 1.2 mol of hydroxyl functional groups) of dextran having a weight-average molar mass of 40 kg/mol (Pharmacosmos, degree of polymerization n = 205), are dissolved in water (285 g/L) at 30°C, then $NaBH_4$ (74 mg, 1.95 mmol) is added and the mixture is stirred at 30°C for 2 h. To this solution is added sodium chloroacetate (140 g, 1.2 mol) and the mixture is heated at 65°C for 1 h. 10 N NaOH (200 mL, 2 mol) is then slowly added over 1.5 h and the mixture is stirred at 65°C for 1 h. The mixture is diluted with water (120 mL), cooled to room temperature, neutralized with acetic acid and then purified by ultrafiltration on PES membrane (MWCO 5 kDa) against phosphate buffer pH 7, then water. This intermediate polysaccharide (polysaccharide 4.1.1) is then freeze-dried.

90 g (0.35 mol of glucoside units) of freeze-dried polysaccharide 4.1.1 are dissolved in water (260 g/L) at 65°C, then sodium chloroacetate (204 g, 1.75 mol) is added and the mixture is maintained at 65°C for 1 h. 10 N NaOH (175 mL, 1.75 mol) is then slowly added over 1 h and the mixture is stirred at 65°C for a further 1 h. Another portion of sodium chloroacetate (122 g, 1.05 mol) is then added and the mixture is maintained at 65°C for 0.5 h. 10 N NaOH (105 mL, 1.05 mol) is then slowly added over 1 h and the mixture is stirred at 65°C for a further 1 h. The mixture is diluted with water, cooled to room temperature, neutralized with acetic acid and then purified by ultrafiltration on PES membrane (MWCO 5 kDa) against phosphate buffer pH 7, then water. The polysaccharide 4.1 concentration of the final solution is determined by dry extract, and then an acid/base assay is carried out in order to determine the degree of substitution with methyl carboxylate.

According to the dry extract: [polysaccharide 4.1] = 48.4 mg/g

According to the acid/base assay, degree of substitution with methylcarboxylate ($DS_2$) = 2.1

Polysaccharide 4.2 - Dextran Methyl Carboxylate Sulfonate

To 400 g of the solution of polysaccharide 4.1 obtained above (48.4 mg/g, $DS_2$ = 2.1, 19.4 g, 58.63 mmol of glucoside units) are added HOPO (9.77 g, 87.95 mmol), 3-amino-1-propanesulfonic acid (Homotaurine) (9.79 g, 70.36 mmol), $Et_3N$ (9.81 mL, 70.36 mmol) and EDC (16.86 g, 87.95 mmol) and the reaction mixture is stirred at 25°C for 2 h. Two additional additions of EDC (16.86 g, 87.95 mmol) are performed every 2 h. The mixture is diluted with phosphate buffer pH 7, then purified by ultrafiltration on PES membrane (MWCO 5 kDa) against phosphate buffer pH 7, NaCl (9 g/L in water) and then water. The polysaccharide 4.2 concentration of the final solution is determined by dry extract, and the degree of substitution with homotaurine is determined by $^1H$ NMR in $D_2O$. The final solution is stored at 4°C.
According to the dry extract: [polysaccharide 4.2] = 40.9 mg/g
According to $^1H$ NMR ($D_2O$), degree of substitution with homotaurine ($DS_3$)= 1.15

Polysaccharide 4 - Dextran Methyl Carboxylate Sulfonate and Maleimide

Using a process similar to the one used for the preparation of polysaccharide 1, starting from polysaccharide 4.2 (40.9 mg/g, $DS_2$ = 2.1, $DS_3$ = 1.15, 9.4 g, 20.0 mmol of glucoside units), and with N-(2-aminoethyl) maleimide hydrochloride (1.06 g, 6.0 mmol), polysaccharide 4 is obtained.
According to the dry extract: [polysaccharide 4] = 19.9 mg/g
According to $^1H$ NMR ($D_2O$), degree of substitution with maleimide ($DS_1$)=0.25

**Polysaccharide 5** - Dextran Methyl Carboxylate Ammonio-Sulfonate and Maleimide

Polysaccharide 5.1 - Dextran Methyl Carboxylate Ammonio-Sulfonate

Using a process similar to the one used for the preparation of polysaccharide 4.2, starting from polysaccharide 4.1 (48.4 mg/g, DS2 = 2.1, 7.3 g, 22.11 mmol of glucoside units), and with HOPO (2.95 g, 26.53 mmol), 3-((2-aminoethyl)-dimethylammonio)propane-1-sulfonate (SB) (7.51 g, 26.53 mmol) (synthesized according: L. Yang et al., J. Mater. Chem. B, 2013, 1, 1421), Et3N (7.4 mL, 53.06 mmol) and EDC (3 x 5.09 g, 3 x 26.53 mmol), polysaccharide 5.1 is obtained.
According to the dry extract: [polysaccharide 5.1] = 34.6 mg/g
According to 1H NMR (D2O), degree of substitution with SB (DS3) = 0.7

Polysaccharide 5 - Dextran Methyl Carboxylate Ammonio-Sulfonate and Maleimide

Using a process similar to the one used for the preparation of polysaccharide 1, starting from polysaccharide 5.1 (34.6 mg/g, $DS_2$ = 2.1, $DS_3$ = 0.7, 7.5 g, 16.7 mmol of glucoside units), and with N-(2-aminoethyl) maleimide hydrochloride (885 mg, 5.01 mmol), polysaccharide 5 is obtained.
According to the dry extract: [polysaccharide 5] = 15.0 mg/g
According to $^1H$ NMR ($D_2O$), degree of substitution with maleimide ($DS_1$)=0.22

**Polysaccharide 6** - Dextran Methyl Carboxylate and Cyclooctyne

To 30 g of solution of polysaccharide 4.1 (48.4 mg/g, $DS_2$ = 2.1, 1.45 g, 4.39 mmol of glucoside units), HOPO (244 mg, 2.20 mmol) and DMF (25 mL) are added, and the mixture is cooled to 4°C. To this solution are added 3-amino-1-(11,12-didehydrodibenz[*b*,*f*]azocin-5(*6H*)-yl)-1-propanone ($DBCO-NH_2$) (365 mg, 1.32 mmol) in DMF (5 mL) and EDC (422 mg, 2.20 mmol) and the reaction mixture is stirred between 4°C and 25°C for 2 h. Two additional additions of EDC (422 mg, 2.20 mmol) are performed every 2 h. The mixture is diluted with phosphate buffer pH 7, then purified by ultrafiltration on PES membrane (MWCO 5 kDa) against phosphate buffer pH 7, NaCl (9 g/L in water) and then water. The polysaccharide 6 concentration of the final solution is determined by dry extract, and the degree of substitution with $DBCO-NH_2$ is determined by $^1H$ NMR in $D_2O$. The final solution is stored at -20°C.
According to the dry extract: [polysaccharide 6] = 6.1 mg/g
According to $^1H$ NMR ($D_2O$), degree of substitution with $DBCO-NH_2$ ($DS_1$)=0.28

**Polysaccharide 7** Dextran Methyl Carboxylate and Azide

Using a process similar to the last step used for the preparation of polysaccharide 1 (step from polysaccharide 1.3 to polysaccharide 1), starting from polysaccharide 4.1 (48.4 mg/g, $DS_2$ = 2.1, 6.2 g, 18.8 mmol of glucoside units), and with 11-azido-3,6,9-trioxaundecan-1-amine ($N_3-PEG_3-NH_2$) (1.23 g, 5.64 mmol), polysaccharide 7 is obtained.
According to the dry extract: [polysaccharide 7] = 24.6 mg/g

According to $^{1}H$ NMR ($D_2O$), degree of substitution with N3-PEG3-NH2 ($DS_1$)=0.28

**Example A2** : **Polyethylene glycol derivatives comprising at least two reactive functions**

Commercial Polyethylene glycol (PEG) derivatives functionalized with reactive functions were purchased. The reactive functions include thiols ("PEG-SH"), Azides (PEG-N3) and Alcynes (PEG-DBCO). Linear homo bifunctional and multi-arm homofunctional having different molecular weights and bearing different functions were used and are shown in the following Table 2.

**Table 2:** List of commercial PEG derivatives used

| PEG | Chemical Name *(Supplier)* | Structure |
|---|---|---|
| PEG-SH-1 | 2-arm PEG-thiol *(NOF; Ref. SUNBRIGHT DE-034SH)* | 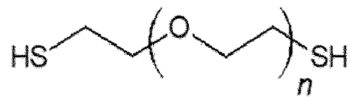<br>Mn (PEG) = 3.4 kg/mol<br>n = 77 |
| PEG-SH-2 | 2-arm PEG-thiol *(Sinopeg Ref. 06020201301)* | 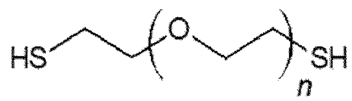<br>Mn (PEG) = 1 kg/mol<br>n = 23 |
| PEG-SH-3 | 4-arm PEG-thiol, pentaerythritol core *(NOF; Ref. SUNBRIGHT PTE-050SH)* | 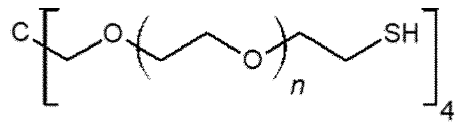<br>Mn (PEG) = 5.2 kg/mol<br>n = 30 |
| PEG-SH-4 | 4-arm PEG-thiol, pentaerythritol core *(Jenkem Ref. 4ARM-SH-20K)* | 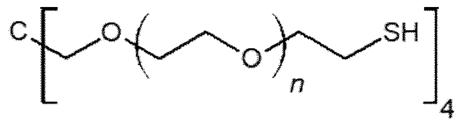<br>Mn (PEG) = 20 kg/mol<br>n = 114 |
| PEG-SH-5 | 4-arm PEG-thiol, pentaerythritol core *(Sinopeg Ref. 06020701315)* | 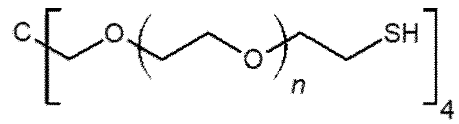<br>Mn (PEG) = 40 kg/mol<br>n = 227 |
| PEG-N3-1 | 4-arm PEG-azide, pentaerythritol core *(Jenkem Ref. 4ARM-AZIDE-20K)* | 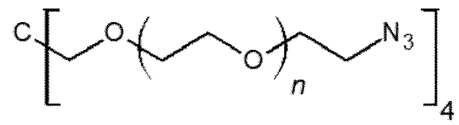<br>Mn (PEG) = 20 kg/mol<br>n = 114 |

(continued)

| PEG | Chemical Name *(Supplier)* | Structure |
|---|---|---|
| PEG-DBCO-1 | 4-arm PEG-DBCO (Dibenzocyclooctyne), pentaerythritol core *(Jenkem Ref. 4ARM-DBCO-20K)* | 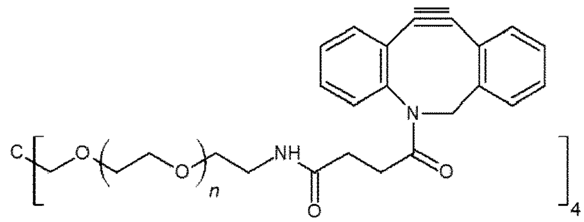 Mn (PEG) = 20 kg/mol<br>n = 114 |

This PEG derivatives correspond to the precursors of the -L of Formula I.

**Part B** - **BIOLOGY**

**Example B1: Primary rat islet isolation**

Pancreatic islets were isolated from male Wistar or Lewis rats (approximative weight: 300g) following a similar method as described in A Pratical Guide to Rodent Islet Isolation and Assessment, Carter et al. Biological Procedures Online 2009.

Briefly, pancreases were perfused with Collagenase injected via the common bile duct. After perfusion, pancreata were excised and the digestion was performed at 37°C for 10 min. The islets were then purified through density gradient centrifugation. Purified islets were cultured in non-adherent culture flasks, in DMEM medium (Gibco) supplemented with 10% Foetal Bovine Serum, 2g/L glucose and 1% Penicillin/Streptomycin at 37°C and 5% CO2. Culture medium was replaced every 2-3 days.

**Example B2: Islet equivalent counting**

To normalize the quantity of islets used in each experiment, islets were counted to determine the islet equivalent count (IEQs). One IEQ corresponds to the volume of a perfectly spherical islet with a diameter of 150 μm. During counting, a multiplicative factor was applied to each islet depending on its size. This mathematical compensation for islet varying diameters allows for normalization between islet preparations (See NIH CIT Consortium Chemistry Manufacturing Controls Monitoring Committee; Purified Human Pancreatic Islet: Qualitative and Quantitative Assessment of Islets Using Dithizone (DTZ): Standard Operating Procedure of the NIH Clinical Islet Transplantation Consortium. CellR4 Repair Replace Regen Reprogram).

Two 50 μL samples from each islet batch were counted on a glass slide with a 50 μm grid. Islets were categorized by size according to Table 4.

**Table 4:** Multiplication factor of islet size for islet equivalent determination

| Islet size | Multiplicative factor |
|---|---|
| 50-100 μm | 1/6 |
| 100-150 μm | 1/1.5 |
| 150-200 μm | 1.7 |
| 200-250 μm | 3.5 |
| 250-300 μm | 6.3 |

The islet equivalent count was determined by averaging the count results from two independent samples.

**Part C - PHYSICO-CHEMISTRY**

**Example C1A: Preparation of solutions of concentrated polysaccharide functionalized with maleimide (Mal) groups**

A concentrated polysaccharide solution was prepared by weighing the appropriate weight of a sterile freeze-dried polysaccharide obtained according to part A1 and adding the appropriate weight of sterile deionised water. The solution was placed on an orbital shaker overnight at 70 rpm for complete solubilization. The pH of the solution was adjusted to pH 4 by addition of concentrated HCl before sterile filtration (0.22 μm). The mass concentration of the solution of polysaccharide (mg/g) was determined by dry exact. The volume concentration of the solution of polysaccharide (mg/mL) was determined by density measurements, weighing three times 100 μL of solution. The solution was frozen at -20°C until being used.

**Example C1B: Preparation of solutions of concentrated polysaccharide functionalized with vinyl sulfone (VS) or DBCO groups**

A concentrated polysaccharide solution was prepared by weighing the appropriate weight of a sterile freeze-dried polysaccharide obtained according to part A1 and adding the appropriate weight of sterile deionized water. The solution was placed on an orbital shaker overnight at 70 rpm for complete solubilization. The pH of the solution was adjusted to pH 7.4 by addition of NaOH before sterile filtration (0.22 μm). The mass concentration of the solution of polysaccharide (mg/g) was determined by dry extract. The volume concentration of the solution of polysaccharide (mg/mL) was determined by density measurements, weighing three times 100 μL of solution. The sterile solution was frozen at -20°C until being used.

**Example C2: Preparation of solutions of concentrated PEG derivative**

A concentrated solution of PEG (from the list according to table 2) was prepared by weighing the appropriate weight of a PEG powder and adding the appropriate weight of sterile deionized water. The solution was placed on roller shaker at 15 rpm for 2 h for complete solubilization before sterile filtration (0.22 μm). The mass concentration of the PEG solution (mg/g) was determined by dry extract. The volume concentration of the PEG solution (mg/mL) was determined by density measurements, weighing three times 100 μL of solution. The sterile solution was frozen at -20°C until being used.

**Example C3: Preparation of solution of concentrated sodium hyaluronate**

A concentrated solution of sodium hyaluronate (Pharma Grade 150 supplied by Novamatrix) was prepared by weighing the appropriate weight of sodium hyaluronate powder and adding the appropriate weight of sterile deionized water. The solution was placed on roller shaker at 10 rpm overnight to complete solubilization before sterile filtration (0.22 μm).

**Example C4A: Hydrogels preparation**

The preparation of the hydrogels was made in an aseptic environment.

Polysaccharide and PEG derivatives concentrated sterile solutions prepared according to example C1A or C1B and example C2, respectively, were adjusted with a concentrated NaCl solution to obtain isotonic stock solutions (300 mOsm/kg) and equilibrated either at room temperature (20-25°C) or at 4°C. Optionally concentrated solution of polysaccharide bearing VS or DBCO groups was supplemented by a tris buffer at pH 7.4 or pH 8.

A concentrated solution of PEG/Hyaluronate was prepared by mixing a concentrated solution of PEG prepared according to example C2 and a concentrated solution of sodium hyaluronate prepared according to example C3.

A concentrated solution of PEG or PEG/Hyaluronate was added to a concentrated solution of polysaccharide in a 2 mL Eppendorf. The volume ratio of the PEG solution to the polysaccharide solution was 70:30 (%:%) or 80:20 (%:%) when hyaluronate is added to the PEG solution. The solutions were mixed with a pipette and a controlled volume of the mixture was introduced in a circular silicone isolator adhering to a glass slide. Different molded hydrogel geometries were prepared.

**Table 5:** Molded hydrogel geometries conditions.

| Geometry | Silicone Isolator Diameter (mm) | Silicone Isolator Thickness (mm) | Hydrogel Volume (μL) |
|---|---|---|---|
| C4A-1 | 4.5 | 0.5 | 10 |
| C4A-2 | 4.5 | 1.6 | 25 |
| C4A-3 | 9 | 0.5 | 32 |
| C4A-4 | 9 | 1.6 | 100 |
| C4A-5 | 10 | 0.5 | 50 |
| C4A-6 | 13 | 0.5 | 66 |
| C4A-7 | 20 | 0.5 | 160 |

Crosslinking leading to gelation was carried out for 1 h at room temperature (20-25°C) or at 37°C. The hydrogel was unmolded and introduced in a Tris 150 mM /NaCl 30 mM / Cystein 10 mM solution at pH 8 or in PBS at pH 7.4 for 1h at 37°C.

The hydrogel was rinsed with PBS solution without cysteine and further immersed in the PBS solution overnight at 37°C. The hydrogel piece was then stored in PBS solution at 4°C until being used.

**Example C5: Hydrogel compositions**

Different hydrogel compositions were prepared according to the protocol described in Example C3 (Table 6). Concentrations of the two reactive groups (maleimide (Mal) or vinylsulfone (VS) reacting with thiol (SH) or alcyne (DBCO) reacting with azide (N3)) and polymers (polysaccharide and PEG derivatives) correspond to the final concentration upon mixing of the polymer solutions.

| *Example* | *Polysaccharide* | *[PEG]* | *Mal :SH or VS :SH or DBCO :N3 (mM:mM)* | *[Polysaccharide] (mg/mL)* | *pH* | *Tris Buffer (mM)* | *[PEG] (mg/mL)* | *Sodium Hyaluronate* | *Mixing Temperature (°C)* | *Gelling temperature (°C)* | *Cystein 10 mM bath* |
|---|---|---|---|---|---|---|---|---|---|---|---|
| C5-1 | 1 | PEG-SH-4 | 5 :5 | 7.8 | 4 | - | 26.4 | - | 4 | 20-25 | Yes |
| C5-2 | 1 | PEG-SH-4 | 5 :5 | 7.8 | 4 | - | 26.4 | 1.5 | 4 | 20-25 | Yes |

| Example | Polysaccharide | [PEG] | Mal :SH or VS :SH or DBCO :N3 (mM:mM) | [Polysaccharide] (mg/mL) | pH | Tris Buffer (mM) | [PEG] (mg/mL) | Sodium Hyaluronate | Mixing Temperature (°C) | Gelling temperature (°C) | Cystein 10 mM bath |
|---|---|---|---|---|---|---|---|---|---|---|---|
| C5-3 | 1 | PEG-SH-4 | 10 :10 | 15.7 | 4 | - | 52.8 | - | 4 | 20-25 | Yes |
| C5-4 | 1 | PEG-SH-4 | 10 :10 | 15.7 | 4 | - | 52.8 | 1 | 4 | 20-25 | Yes |
| C5-5 | 1 | PEG-SH-4 | 10 :10 | 15.7 | 4 | - | 52.8 | 1.25 | 4 | 20-25 | Yes |
| C5-6 | 1 | PEG-SH-4 | 10 :10 | 15.7 | 4 | - | 52.8 | 1.5 | 4 | 20-25 | Yes |
| C5-7 | 1 | PEG-SH-4 | 10 :10 | 15.7 | 4 | - | 52.8 | 2.5 | 4 | 20-25 | Yes |
| C5-8 | 1 | PEG-SH-4 | 14 :14 | 22 | 4 | - | 74 | - | 4 | 20-25 | Yes |
| C5-9 | 1 | PEG-SH-4 | 14 :14 | 22 | 4 | - | 74 | 1 | 4 | 20-25 | Yes |
| C5-10 | 1 | PEG-SH-5 | 5 :5 | 7.8 | 4 | - | 52.5 | - | 4 | 20-25 | Yes |
| C5-11 | 1 | PEG-SH-5 | 5 :5 | 7.8 | 4 | - | 52.5 | 1.5 | 4 | 20-25 | Yes |
| C5-12 | 6 | PEG-N3-1 | 7 :7 | 9.9 | 7.4 | 6 | 36.8 | - | 4 | 20-25 | No |
| C5-13 | 6 | PEG-N3-1 | 7 :7 | 9.9 | 7.4 | 6 | 36.8 | 1.25 | 4 | 20-25 | No |
| C5-14 | 6 | PEG-N3-1 | 9 :9 | 12.8 | 7.4 | 6 | 47.6 | - | 4 | 20-25 | No |
| C5-15 | 6 | PEG-N3-1 | 9 :9 | 12.8 | 7.4 | 6 | 47.6 | 1.25 | 4 | 20-25 | No |

**Table 6:** Compositions of various hydrogels made of polysaccharide and PEG derivatives and optionally incorporating sodium hyaluronate.

Solid disc-shaped hydrogel pieces were obtained. The hydrogels pieces were easily unmolded and handled with tweezers for characterization.

**Table 6a:** Structures of the hydrogels

| Polysaccharide + PEG-SH | Structure |
|---|---|
| Polysaccharide 1 + PEG-SH-4 | 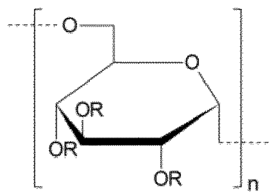<br>Mw (Dextran) = 40 kg/mol<br>n = 205<br>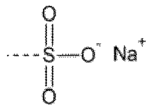 or 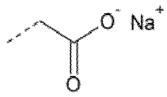 or<br>R = H or<br>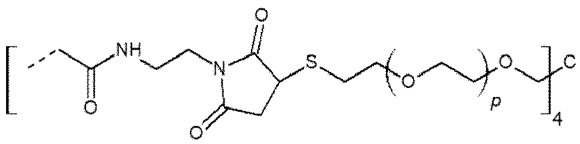<br>p = 114<br>$DS_3$ (sulfate) = 1.5<br>$DS_2$ (methylcarboxylate) = 0.8<br>$DS_1$ (succinimide derivative) = 0.25 |
| Polysaccharide 1 + PEG-SH-5 | 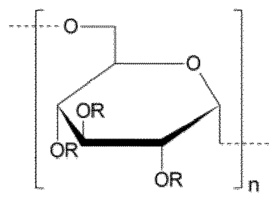<br>Mw (Dextran) = 40 kg/mol<br>n = 205<br>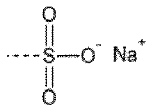 or 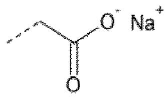 or<br>R = H or<br>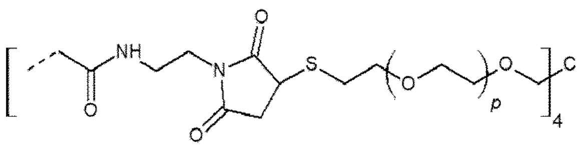<br>p = 227<br>$DS_3$ (sulfate) = 1.5<br>$DS_2$ (methylcarboxylate) = 0.8<br>$DS_1$ (succinimide derivative) = 0.25 |

(continued)

| Polysaccharide + PEG-SH | Structure |
|---|---|
| Polysaccharide 6 + PEG-N3-1 | 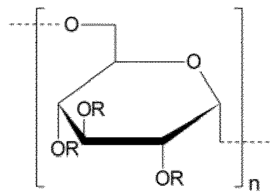<br>Mw (Dextran) = 40 kg/mol<br>n = 205<br>R = H or 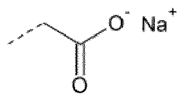 or<br>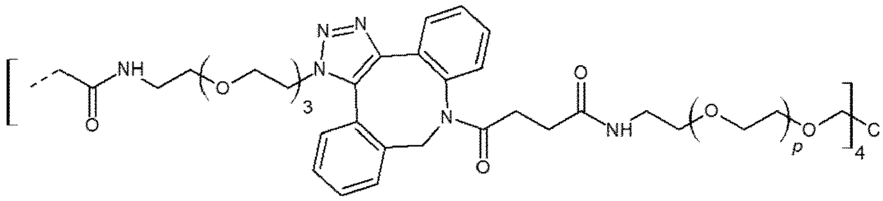<br>or<br>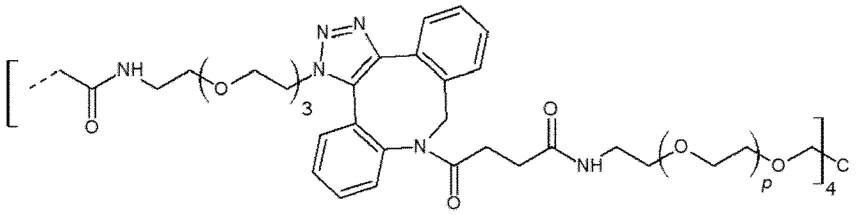<br>p = 114<br>$DS_2$ (methylcarboxylate) = 2.1<br>$DS_1$ (triazole derivatives (mixture of 2 isomers)) = 0.28 |

**Example C6: Hydrogel rheological characterization**

Oscillatory shear test was carried out with a rotational rheometer (AR2000, TA instrument) equipped with a cone plate geometry. The crosslinking leading to gelation was done "in situ", meaning that drops of polysaccharide and PEG concentrated solutions were introduced between cone one and plate and mixed by rotation of the geometry before starting oscillations measurements. Oscillation time sweep tests were carried out at 25°C or 37°C, with a constant strain of 0.1% and constant oscillation frequency of 1 Hz. The storage modulus G' (i.e. elastic modulus) and Tan δ (ratio G'/G") values were reported at 1600 s in the plateau region of the measure of (G',G") as a function of time.

| Example | Hydrogel composition | Polysaccharide | [PEG] | Mal :SH or VS :SH or DBCO :N3 (mM:mM) | pH | Tris Buffer (mM) | Sodium Hyaluronate (mg/ml) | Mixing Temperature (°C) | Gelling temperature (°C) | Gelation onset (min) (time G' > 1 Pa) | G' (kPa) 1h | Tan δ(G' /G'') |
|---|---|---|---|---|---|---|---|---|---|---|---|---|
| C6-1 | C5-3 | 1 | PEG-SH-4 | 10 :10 | 4 | - | 0 | 4 | 20-25 | > 1 | 10.5 | <0.01 |
| C6-2 | C5-5 | 1 | PEG-SH-4 | 10 :10 | 4 | - | 1.25 | 4 | 20-25 | > 1 | 12.2 | <0.01 |
| C6-3 | C5-7 | 1 | PEG-SH-4 | 10 :10 | 4 | - | 2.5 | 4 | 20-25 | > 1 | 13.2 | <0.01 |
| C6-4 | C5-12 | 6 | PEG-N3-1 | 7 :7 | 7.4 | 6 | - | 4 | 20-25 | <1 | 6.3 | <0.01 |
| C6-5 | C5-13 | 6 | PEG-N3-1 | 7 :7 | 7.4 | 6 | 1.25 | 4 | 20-25 | <1 | 4.7 | <0.01 |
| C6-6 | C5-14 | 6 | PEG-N3-1 | 9 :9 | 7.4 | 6 | - | 4 | 20-25 | <1 | 10.6 | <0.01 |
| C6-7 | C5-15 | 6 | PEG-N3-1 | 9 :9 | 7.4 | 6 | 1.25 | 4 | 20-25 | < 1 | 9.6 | <0.01 |

***Table 7:*** *Results of hydrogel rheological characterization.*

Hydrogels present low values of Tan δ, meaning that G' was much higher than G" which is a typical property of chemically cross-linked hydrogels behaving as solid elastic materials (see Polysaccharide Hydrogels: Characterization and Biomedical Applications, 2016 Pan Stanford Publishing Pte. Ltd.; Chapter 3, page 97).

The increase of the concentration in reactive groups leads to an increase of the value of elastic modulus G'.

This can allow a fine tuning of the crosslinking leading to gelation speed which could be convenient as fast gelation could be beneficial to avoid cells sedimentation whereas slow gelation could be beneficial for polymer mix casting before gelation.

Gelation properties are maintained in presence of non-reactive sodium hyaluronate.

This can allow a further tuning of hydrogel composition to avoid cells sedimentation, even for slow geling compositions.

**Example C7: Hydrogel swelling and water content**

The hydrogel piece was weighed right after unmoulding (w0) and after overnight swelling (wovernight) in the PBS solution. The swelling ratio was defined as the mass ratio wovernight/w0. The water content of the hydrogel was deducted from the measurement of hydrogel mass in the swollen state and the control of polymer precursors concentrations implemented to synthetize the hydrogel.

| *Example* | *Hydrogel composition* | *Polysaccharide* | *PEG* | *Hyaluronate (mg/mL)* | *Mal :SH or VS :SH or* | *swelling ratio* | *Water content (weight%)* |
|---|---|---|---|---|---|---|---|
| C7-1 | C5-3 | 1 | PEG-SH-4 | 0 | 10 :10 | 0.9 | 93 |
| C7-2 | C5-5 | 1 | PEG-SH-4 | 1.25 | 10 :10 | 1.3 | 95 |
| C7-3 | C5-12 | 6 | PEG-N3-1 | 0 | 7 :7 | 0.8 | 94 |
| C7-4 | C5-13 | 6 | PEG-N3-1 | 1.25 | 7 :7 | 1 | 95 |
| C7-5 | C5-14 | 6 | PEG-N3-1 | 0 | 9 :9 | 0.9 | 93 |
| C7-6 | C5-15 | 6 | PEG-N3-1 | 1.25 | 9 :9 | 1 | 94 |

***Table 8:*** *Hydrogel swelling and water content.*

Hydrogels contain high water content.

**Example C8: Hydrogel stability at 37°C in physiological medium**

Hydrogel pieces were stored in PBS at pH 7.4 or in Serum (FBS Foetal Bovine Serum) at 37°C and weighed at different periods of time. Disc-shaped hydrogels with a diameter of 9 mm and a thickness of 1.6 mm were tested.

***Table 9:*** *Hydrogel stability in physiological media*

| *Example* | *Hydrogel composition* | *media* | *number of hydrogels weighed* | *Gel Mass to* | *Time at 37°C (days)* | *Gel Mass timing t (mg)* |
|---|---|---|---|---|---|---|
| C8-1 | C5-3 | PBS | 2 | 134 ± 2 | 34 | 134 ± 2 |
| | | Serum | 2 | 140 ± 2 | 16 | 133 ± 2 |
| | | | | 137 ± 1 | 34 | 137 ± 1 |
| C8-2 | C5-5 | PBS | 2 | 138 ± 1 | 34 | 138 ± 1 |
| | | Serum | 2 | 143 ± 3 | 16 | 139 ± 3 |
| | | | | 134 ± 7 | 34 | 132± 7 |

Hydrogels were retrieved intact and their mass does not evolve significantly upon storage at 37°C in physiological medium. Hydrogel swelling (mass increase) or dissolution (mass decrease) would be expected in case of network structure modification in case of hydrolysis side reaction for example. This shows that the hydrogel was stable under physiological conditions.

**Example C9: Encapsulation of macromolecular probes within hydrogel**

Commercial Fluorescent Dextran-FITC 3 kDa and Dextran-FITC 70 kDa were each solubilized in water to obtain concentrated stock solutions.

Polysaccharide DMCMal and PEG-SH concentrated sterile solutions prepared according to example C1 and C2, respectively, were equilibrated at 4°C. For DMCVS based hydrogel, polysaccharide DMCMal and PEG-SH concentrated solutions prepared according to example C1A and C2, respectively, were equilibrated at 20-25°C.

The solution of polysaccharide DMCMal or DMCVS was mixed with a fluorescent dextran. 100 μL of a concentrated solution of PEG-SH was added to 100 μL of a concentrated solution of polysaccharide DMCMal or DMCVS and fluorescent dextran. The solutions were mixed with a pipette and 200 μL of the latter mixture was introduced in a rectangular silicone mould (IBIDI 12x7.75 mm). Crosslinking leading to gelation was carried out for 1 h at room temperature (20-25°C) for DMCMal based hydrogel or 1h at 37°C for DMCVS based hydrogel.

The hydrogel piece was unmoulded, introduced in a well (12 wells multi-plate) and immersed with 1.3 mL of a buffer solution of Tris (200 mM)/NaCl (50 mM) at pH 8 containing the same concentration of encapsulated fluorescent dextran. Hydrogel swelling was performed overnight at 37°C and the supernatant was weighed to estimate the degree of swelling and the quantity (mg) in the hydrogel volume.

Hydrogels were quickly rinsed twice with 1 mL of a buffer solution of Tris (200 mM)/NaCl (50 mM) at pH 8 before release experiment (shown in example C8).

| Example | Polysaccharide | PEG | Mal :SH (mM:mM) | [Polysaccharide] (mg/mL) | pH | [PEG] (mg/mL) | [Hyaluronate] (mg/mL) | Dextran (kda) | [Dextran] (mg/mL) |
|---|---|---|---|---|---|---|---|---|---|
| C9-1 | 1 | PEG-SH-4 | 10 :10 | 16 | 4 | 53 | - | 3 | 1.5 |
| C9-2 | 1 | PEG-SH-4 | 10 :10 | 16 | 4 | 53 | - | 70 | 1.5 |
| C9-3 | 1 | PEG-SH-4 | 10 :10 | 16 | 4 | 53 | 1.5 | 3 | 1.5 |
| C9-4 | 1 | PEG-SH-4 | 10 :10 | 16 | 4 | 53 | 1.5 | 70 | 1.5 |

***Table 10:*** *Compositions of hydrogel with encapsulated fluorescent macromolecular probe*

**Example C10: Release of macromolecular probes within hydrogel**

Hydrogels with encapsulated fluorescent probe as produced in Example C7 were each introduced in a well (12 wells multi-plate) and immersed with 2 mL of a buffer solution of Tris (200 mM)/NaCl (50 mM) at pH 8. The plate was covered with a film and introduced in an oven at 37°C. 200 μL of buffer were sampled at different time point and replaced

by fresh buffer. Fluorescent probe concentration in the samples was determined by fluorescence (fluorescent plate reader SAFAS) using a calibration curve. The cumulative fraction of fluorescent probe released at each time point corresponds to the ratio of the cumulative quantity of fluorescent probe released to the initial quantity of fluorescent probe in the swollen hydrogel.

**Table 11:** Cumulative fraction of macromolecular probes released from the hydrogel at different time points.

| Example | Composition | Time (h) | 0.25 | 0.5 | 1 | 24 |
|---|---|---|---|---|---|---|
| C10-1 | C9-1 | % Dextran 3 kDa released | >10 | >20 | >30 | >40 |
| C10-2 | C9-2 | % Dextran 70 kDa released | <5 | <5 | <5 | <20 |
| C10-3 | C9-3 | % Dextran 3 kDa released | >10 | >20 | >30 | >40 |
| C10-4 | C9-4 | % Dextran 70 kDa released | <5 | <5 | <5 | <20 |

Increasing the size of the macromolecular probe leads to a slower kinetic of release. This shows the permselective property of the hydrogel's network structure.

**Example C11: Determination of mechanical resistance of the hydrogels.**

For compression, a swollen rectangular hydrogel piece as described in example C3 was introduced in a flat glass crystallizer and immersed in PBS. The uniaxial compression was done in NaCl 0.9% at 20-25°C by using a universal mechanical tester apparatus (Zwickroell) equipped with flat compression plates, at a speed of 0.2 mm/min. Initial thickness of the sample was determined from the contact of the plate with the hydrogel, when the force starts to increase. The deformation is defined by the ratio of the compression displacement (mm) and the initial thickness (mm). The deformation at break was determined from the force/displacement curve. The break is defined when a decrease of the force versus displacement was observed.

The Young modulus was determined from the slope of the true strain / deformation curve. The true strain (kPa) corresponds to the ratio of the force (N) to the instantaneous surface area (mm2) of the sample under compression.

| Example | Composition | Polysaccharide | PEG | Mal or VS :SH or DBCO:N3 (mM:mM) | Hyaluronate (mg/mL) | pH | Compression Deformation at break (%) | Compression Young Modulus (kPa) |
|---|---|---|---|---|---|---|---|---|
| C11-1 | C5-3 | 1 | PEG-SH-4 | 10:10 | 0 | 4 | >60 | >30 |
| C11-2 | C5-5 | 1 | PEG-SH-4 | 10:10 | 1.25 | 4 | >60 | >30 |

| Example | Composition | Polysaccharide | PEG | Mal or VS :SH or DBCO:N3 (mM:mM) | Hyaluronate (mg/mL) | pH | Compression Deformation at break (%) | Compression Young Modulus (kPa) |
|---|---|---|---|---|---|---|---|---|
| C11-3 | C5-12 | 6 | PEG-N3-4 | 7:7 | 0 | 7.4 | >60 | >30 |
| C11-4 | C5-13 | 6 | PEG-N3-4 | 7:7 | 1.25 | 7.4 | >60 | >30 |
| C11-5 | C5-14 | 6 | PEG-N3-4 | 9:9 | 0 | 7.4 | >60 | >50 |
| C11-6 | C5-15 | 6 | PEG-N3-4 | 9:9 | 1.25 | 7.4 | >60 | >50 |

**Table 13:** Mechanical resistance of hydrogels

The hydrogels according to the invention exhibit very good mechanical resistances features combining deformability and stiffness suitable for surgical implantation.

**Example C12: Alkaline treatment of polyester surgical net.**

Warp knit Polyester surgical net fabric type PETKM3002 (1x0.9mm pore size) supplied by SurgicalMeshTM was treated in NaOH 1M for 5 hours at 70°C and rinsed with deionized water and ethanol 96%. The treatment led to an increased hydrophilicity of the net fabric leading to an improved wetting with aqueous solutions of polymers constituting the hydrogel.

**Example C13: Fabrication of Ring Net constructs**

Biocompatible PDMS sheets supplied by Grace Biolabs or Interstate Speciality Product were cut in a form of a square incorporating a circular empty disc using a stainless steel punch.

A part of the treated polyester surgical net described in example C12 was introduced in between two square PDMS pieces: The two PDMS pieces and the surgical net were glued together with biocompatible silicone glue (Silbione MED ADH 4200 supplied by Elkem). The circular empty discs were aligned, and the surgical net was kept tense during gluing.

Finally, the square construct was cut with a strainless steel punch to obtain the final object constituted by two PDMS rings sandwiching a surgical net and glued together.

The pieces were washed with a solution of poloxamer F127 at 1% and rinsed with water before steam sterilization.

**Example C14: Ring net constructs of different dimensions**

Different Ring Net constructs were fabricated according to example C13 by varying parameters such as internal/external diameter and thickness of the PDMS rings and the thickness of the glue.

**Table 14:** Dimensions of Ring Net constructs.

| Example | PDMS Ring thickness (each part) (μm) | Interior Ring diameter (mm) | Exterior Ring Diameter (mm) | Net type | Net thickness (μm) | Glue thickness (μm) | Total thickness (μm) |
|---|---|---|---|---|---|---|---|
| C14-1 | 430 | 12 | 16 | 3002 | 230 | ~200 | 1250 |

(continued)

| Example | PDMS Ring thickness (each part) (μm) | Interior Ring diameter (mm) | Exterior Ring Diameter (mm) | Net type | Net thickness (μm) | Glue thickness (μm) | Total thickness (μm) |
|---|---|---|---|---|---|---|---|
| C14-2 | 250 | 12 | 16 | 3002 | 230 | ~200 | 900 |
| C14-3 | 250 | 16 | 20 | 3002 | 230 | ~200 | 900 |
| C14-4 | 250 | 36 | 44 | 3002 | 230 | ~200 | 900 |
| C14-5 | 150 | 12 | 16 | 3002 | 230 | ~200 | 700 |
| C14-6 | 150 | 12 | 16 | 3002 | 230 | ~100 | 600 |
| C14-7 | 100 | 12 | 16 | 3002 | 230 | ~100 | 500 |

**Exemple C15: Hydrogel / Ring Net composites compositions**

Hydrogel compositions prepared according to example C4 and described in example C5 were incorporated in the Ring Net constructs described in example C14. The concentrated polymer solutions were mixed with a pipette and a controlled volume of the mixture was introduced in a ring net construct adhering to a glass slide.

Crosslinking leading to gelation was carried out for 1 h at room temperature (20-25°C) or at 37°C. The Ring Net / Hydrogel composition was then introduced in a Tris 150 mM /NaCl 30 mM / Cystein 10 mM solution at pH 8 or in PBS at pH 7.4 for 1h at 37°C.

The hydrogel was rinsed with PBS solution without cysteine and further immersed in the PBS solution overnight at 37°C. The hydrogel piece was then stored in PBS solution at 4°C until being used.

Different Hydrogel / Ring Net composites compositions were prepared (Table 15):

**Table 15:** Hydrogel / Ring Net composites compositions.

| Example | Hydrogel Composition | Ring Net Construct | Hydrogel Volume (μL) | Cysteine 10 mM bath |
|---|---|---|---|---|
| C15-1 | C5-5 | C14-1 | 220 | Yes |
| C15-2 | C5-3 | C14-2 | 120 | Yes |
| C15-3 | C5-5 | C14-2 | 120 | Yes |
| C15-4 | C5-5 | C14-2 | 140 | Yes |
| C15-5 | C5-5 | C14-3 | 230 | Yes |
| C15-6 | C5-5 | C14-4 | 500 | Yes |
| C15-7 | C5-5 | C14-5 | 110 | Yes |
| C15-8 | C5-13 | C14-6 | 90 | Yes |
| C15-9 | C5-13 | C14-6 | 80 | Yes |
| C15-10 | C5-13 | C14-7 | 90 | Yes |
| C15-11 | C5-13 | C14-7 | 80 | Yes |

Hydrogel/Ring Net composites are easily manipulated with tweezers and are foldable for the need of surgical implantation. Moreover, it is possible to fix the ring with sutures.

The hydrogel volume can be adjusted with the internal diameter and the thickness or the ring net construct. For the same ring net construct the hydrogel volume can be adjusted to control the convexity of the hydrogel above the ring level.

**Example C16: Encapsulation of insulin producing cell islets in Hydrogels**

Cell islets encapsulation was made in an aseptic environment.

Polysaccharide and PEG concentrated sterile solutions prepared according to examples C1 or C1A and C2, respectively, were adjusted with a concentrated NaCl solution in order to obtain isotonic stock solutions (300 mOsm/kg).

Solutions were equilibrated either at room temperature (20-25°C or at 4°C)

A concentrated mixture of PEG and islets was prepared by mixing equal volumes of isotonic PEG solution or PEG / Sodium hyaluronate solution (prepared according to example C2, C3 and C4A) and islets suspension (prepared according to example B1). Then the mixture containing PEG and islets was gently mixed with an isotonic concentrated solution of polysaccharide by a 70:30 Volume:Volume proportion of islets suspension/PEG : polysaccharide or 80:20 Volume:Volume proportion of islets suspension/ PEG/Hyaluronate .: polysaccharide.

The solutions were gently mixed with a pipette and a controlled volume of the mixture was introduced in a circular silicone isolator (from Grace Biolab) adhering to a glass slide or in a Ring Net construct. Different hydrogel sizes were made by using different silicone molds (Table 5) or ring net constructs (Table 15)

Crosslinking leading to gelation was carried out for 1 h at room temperature (RT, 20-25°C) or in an oven at a controlled temperature such as 20, 25 or 37°C. The hydrogel disc or hydrogel / ring net incorporating islets was introduced in a Tris 150 mM / NaCl 30 mM / Cystein 10 mM solution at pH 8 (Mal or VS : SH cross-linking) or in culture medium for 15 min at room temperature (DBCO:N3 cross-linking). Hydrogels were then immersed in culture medium containing 10 mM cysteine for 1 h at 37°C or in culture medium without cysteine. After 1 h the culture medium containing cysteine was removed and replaced by culture medium. Sterile hydrogels containing cells were stored at 37°C and 5% CO2 before further in vitro testing or in vivo implantation.

Different hydrogel / Islets compositions were prepared according to this process.

| Example | Polysaccharide | PEG | Mal or VS :SH or DBCO:N3 (mM:mM) | Sodiulm Hyaluronate 1500 kDa (mg/mL) | pH | Gelation Temperature ( °C) | Islets type | islets density (islets equivalent/mL) | Implant Geometry | Hydrogel Volume (µL) |
|---|---|---|---|---|---|---|---|---|---|---|
| C16-1A | 1 | PEG-SH-4 | 10:10 | 0 | 4 | RT | Wistar rat | 34, 000 | Ring Net C14-2 | 120 |
| C16-1B | 1 | PEG-SH-4 | 10:10 | 0 | 4 | RT | Wistar rat | 34, 000 | Disc C4A-1 | 10 |
| C16-2A | 1 | PEG-SH-4 | 10:10 | 1.25 | 4 | RT | Wistar rat | 16, 000 | Ring Net C14-2 | 120 |
| C16-2B | 1 | PEG-SH-4 | 10:10 | 1.25 | 4 | RT | - | 0 | Ring Net C14-2 | 120 |
| C16-3A | 1 | PEG-SH-4 | 10:10 | 1.5 | 4 | RT | Wistar rat | 7,000 | Ring Net C14-2 | 120 |

| Example | Polysaccharide | PEG | Mal or VS :SH or DBCO:N3 (mM:mM) | Sodiulm Hyaluronate 1500 kDa (mg/mL) | pH | Gelation Temperature ( °C) | Islets type | islets density (islets equivalent/mL) | Implant Geometry | Hydrogel Volume (µL) |
|---|---|---|---|---|---|---|---|---|---|---|
| C16-3B | 1 | PEG-SH-4 | 10:10 | 1.5 | 4 | RT | Wistar rat | 7,000 | Ring Net C14-3 | 230 |
| C16-4A | 1 | PEG-SH-4 | 14:14 | 1 | 4 | RT | Wistar Rat | 40, 000 | Disc C4A-1 | 10 |
| C16-4B | 1 | PEG-SH-4 | 14:14 | 1 | 4 | RT | Lewis | 40, 000 | Disc C4A-1 | 10 |
| C16-5 | 1 | PEG-SH-4 | 10:10 | 1.25 | 4 | 20 | Wistar rat | 36, 000 | Ring Net C14-5 | 110 |
| C16-6 | 1 | PEG-SH-5 | 5.5 | 0 | 4 | RT | Wistar rat | 10,000 | Disc C4A-2 | 25 |
| C16-7 | 1 | PEG-SH-5 | 5.5 | 1 | 4 | RT | Wistar rat | 10,000 | Disc C4A-2 | 25 |
| C16-8 | 1 | PEG-SH-5 | 5.5 | 1.5 | 4 | RT | Wistar rat | 10,000 | Disc C4A-2 | 25 |
| C16-9 | 1 | PEH-SH-4 | 10:10 | 1.25 | 4 | RT | Wistar rat | 16,000 | Disc C4A-2 | 25 |
| C16-10 | 1 | PEH-SH-4 | 10:10 | 1.5 | 4 | RT | Wistar rat | 16,000 | Disc C4A-2 | 25 |

**Table 16:** Hydrogel compositions incorporating islets of different nature at different density.

**Example C17: Spatial homogeneity of encapsulated cells in hydrogel thickness**

Hydrogels discs incorporating islets were prepared as described in example C16. Hydrogel pictures were taken from side to evaluate islets homogeneity trough the thickness of the hydrogel. Homogenity is achieved when the islets are located within the whole thickness of the hydrogel and not essentially localized on one side of the hydrogel.

| Example | Composition | Homogeneity |
|---|---|---|
| C17-1 | C16-6 | No |
| C17-2 | C16-7 | No |
| C17-3 | C16-8 | Yes |
| C17-4 | C16-9 | Yes |
| C17-5 | C16-10 | Yes |

The addition of sodium hyaluronate permit to obtain homogeneity of the spatial localization of the islets in the thickness of the hydrogel.

**Part D - BIOLOGICAL EVALUATIONS OF HYDROGELS**

**Example D1: *In vitro* viability and functionality of encapsulated islets in C16-4A and C16-4B**

*a) Rat primary islet encapsulation*

Rat primary islets (from Wistar or Lewis rat donors) were encapsulated 1 day after isolation, as described in example C16-4A and C16-4B and maintained in culture in vitro.

*b) In vitro viability evaluation*

The viability of encapsulated rat primary islets was evaluated 9 days after encapsulation by Live/Dead staining (ThermoFisher).

Briefly, samples were first washed in Phosphate Buffer Saline (PBS) and then incubated for 60min in PBS supplemented with 2μM calcein-AM and 8μM ethidium bromide. They were finally washed in PBS and imaged using epifluorescence microscopy. Quantification of the viability was performed by segmenting the images. The integrated intensity (sum of area pixel intensity) in the green channel, V (live cells), and the integrated intensity in the red channel, D (dead cells), were calculated. A viability ratio was obtained by calculating $\frac{V}{(V+D)}$.

*c) In vitro functionality evaluation*

To evaluate the functionality of encapsulated rat primary islets, a perifusion experiment was performed. At the selected date after encapsulation, samples were introduced in a perifusion chamber. Chambers were then simultaneously perfused following the protocols described in Table 17.

Flow-through buffer was collected, and insulin was quantified with a qualified ELISA specific for insulin.

The Secretion Index for each sample was calculated as the ratio of the average insulin concentration measured during the stimulation step over the average insulin concentration measured during the 1st basal secretion step.

**Table 17:** Perifusion conditions

| Step | Solution | Duration (min) | Flow Collection Frequency |
|---|---|---|---|
| Equilibration - G3 | Krebs buffer + 3mM Glucose (G3) | 50 | 100μL/min No collection |
| 1st Basal secretion - G3 | | 12 | 100μL/min 1 well/2min |
| Stimulation - G17 | Krebs buffer + 17mM Glucose (G17) | 36 | |
| 2nd Basal secretion - G3 | Krebs buffer + 3mM Glucose (G3) | 12 | |
| Stimulation - KCl | Krebs buffer + 25mM KCl | 6 | |
| 3rd Basal secretion - G3 | Krebs buffer + 3mM Glucose (G3) | 6 | |

*d) Rat primary islet viability and functionality is maintained in C16-4A and C16-4B*

Functionality and viability of rat primary islets encapsulated in C16-4A and C16-4B were evaluated 8 and 9 days after encapsulation, respectively (Table 18). The results were pooled.

***Table 18:*** *Viability and functionality of encapsulated rat primary islets. Errors are standard deviation N = 4 (C16-4A N=2 and C16-4B N=2)*

| Example | Viability Day 9 | Functionality Day 8 Secretion index |
|---|---|---|
| C16-4A and C16-4B | 87.2% +/- 6.9 | 3.45 +/- 1.68 |

The viability score of rat primary islets encapsulated in C16-4A and C16-4B was high at day 9: 87.2% +/- 6.9. In addition, the rat primary islets encapsulated in C16-4A and C16-4B maintain their ability to secrete insulin in response to glucose stimulation: secretion index = 3.45 +/- 1.68.

In conclusion, rat primary islet viability and functionality are maintained in C16-4A and C16-4B.

**Example D2: Long-term *in vitro* functionality of islets encapsulated in C16-1A** *Rat primary islet encapsulation*

Rat primary islets were encapsulated 1 day after isolation, as described in example C16 and maintained in culture in vitro for 28 days.

*e) In vitro functionality evaluation*

To evaluate the in vitro functionality of encapsulated rat primary islets, GSIS (Glucose Stimulated Insulin Secretion) experiments were performed.

Briefly, encapsulated islets were first washed 3 times in Krebs buffer containing 0.1% BSA en 3mM of glucose (Solution G3) (Table 19). Insulin secretion was then equilibrated by incubating the encapsulated islets in Solution G3 for 2 x 30 min at 37°C. Basal insulin secretion was obtained by incubating encapsulated islets in Solution G3 for another 60 min at 37°C. At the end of this incubation, media were collected (Basal insulin secretion samples). Encapsulated islets were then stimulated in Krebs buffer containing 0.1% BSA and 17mM of glucose (Solution G17) for 60 min at 37°C. At the end of the incubation, media were collected (Stimulated insulin secretion samples).

**Table 19:** GSIS buffers and conditions.

| Step | Solution | Duration (min) | Insulin quantification |
|---|---|---|---|
| Wash | Krebs buffer + 3mM Glucose (Solution G3) | 2x 10min | Not performed |
| Equilibration - G3 | | 2x 30 min | |
| Basal secretion - G3 | | 60 min | Basale insulin secretion |
| Stimulation - G17 | Krebs buffer + 17mM Glucose (Solution G17) | 60min | Stimulated insulin secretion |

Insulin levels in these samples were the quantified using a qualified ELISA assay specific for insulin. Secretion index was calculated as the ratio of insulin concentrations measured between Stimulation and Basal secretion steps.

*f) The long term functionality of rat primary islets is maintained when encapsulated in C16-1A*

Functionality rat primary islets encapsulated in C16-1A was evaluated 28 days after encapsulation. The secretion index was 2.9 showing that the functionality of rat primary islets is maintained in C16-1A.
In conclusion, the long-term functionality of rat primary islets is maintained when encapsulated in C16-1A.

**Example D3: *In vitro* functionality of islets encapsulated in C16-5**

*g) Rat primary islet encapsulation*

Rat primary islets were encapsulated 1 day after isolation, as described in example C16-5.

*h) The functionality of rat primary islets is maintained in vitro when encapsulated in C16-5*

In vitro functionality of rat primary islets encapsulated in C16-5 was evaluated as described in Example D2b, 1 day after encapsulation (Table 20).

***Table 20:*** *Functionality of encapsulated rat primary islets. N=1 hydrogel per condition*

| Example | Functionality D1 Secretion index |
|---|---|
| C16-5 | 3.1 |

The secretion indexes were 3.1 and 3.0 respectively, showing that the functionality of encapsulated rat primary islets was maintained in C16-5.
In conclusion, the functionality of rat primary islets is maintained in vitro when encapsulated in C16-5.

**Example D4: *In vivo* tolerance, survival and functionality of islets encapsulated in C16-1A**

*Induction of diabetes*

Diabetes was induced by a single intraperitoneal injection of 75 mg/kg Streptozotocin (Santa Cruz re U-9889). Animals were supplemented with basal insulin via pellets until 5 days before implantation, followed by daily administration of a bolus subcutaneous injection of Lantus (6 U/animal) up to the day of implantation.

*a) Rat primary islets encapsulation*

Wistar rat primary islets were encapsulated 1 day after isolation, as described in example C16-1A.

*b) In vivo implantation*

Wistar rats weighing between 260 g and 310 g at surgery were used. Each animal was implanted with 4 000 IEq.

Anesthesia was performed with isoflurane.

Each animal was placed in the supine position on a warmed pad. The fur was shaved from surgical area with a surgical blade. The surgical site was disinfected with povidone iodine solution.
A 2-3 cm long midline incision was made in the abdomen. The intestines were moved to the left side and covered with saline-soaked gauze to prevent dehydration. The stomach was fully exposed, and the omentum was spread on a humid gauze. One C16-1A implant was placed onto the omentum and covered with it (wrapping technique). Non-resorbable suture stitches (Prolene 6/0) were made on the omentum to maintain the implant in place and avoid their slippage and/or overlapping. Prior to closing the abdomen, 2 mL of physiological saline solution were dispensed into the abdominal cavity to prevent dehydration. The peritoneum with muscular layer was closed with continuous non-resorbable suture (Prolene 4/0), then the skin was sutured (Prolene 4/0) by interrupted single stitches and the incision cleaned with povidone iodine solution.
After surgery, each animal was moved to a recovery area and monitored for recovery from anesthesia until sternal recumbency was achieved. After recovery, the animals were group-housed and observed for general health.

After 4 weeks, the animals were sacrificed. The implantation site was collected; half of the explant from each animal was dissected free from tissues for post-implantation functionality and viability assays, and the other half processed for histology.

*c) Evolution of insulinemia*

Islets' functionality was assessed by specific measurement of rat plasma insulin up to 21 days after implantation. Blood was collected at days 2-, 7-, 14- and 21-post implantation. Plasma was prepared and rat insulin quantified using a qualified specific ELISA immunoassay (Mercodia, Ultrasensitive Rat Insulin ELISA, Ref. 10-1251-01). See Table21.

**Table 21:** Insulin concentrations in plasma of diabetic rats implanted with C16-1A for 28 days. Indicated days are days post implantation. The lower limit of quantification of the ELISA method was defined at 32.0 pM.

| Rat ID | Plasmatic insulin concentration (pM) | | | |
|---|---|---|---|---|
| | Day 2 | Day 7 | Day 14 | Day 21 |
| 129 | 162.2 | 148.9 | 248.3 | 283.8 |
| 130 | 104.1 | 122.4 | 59.2 | 132.1 |
| 133 | 220.7 | 309.2 | 219.0 | 184.5 |

Stable plasmatic insulin levels were detectable for all animals from 2- to 21- days post implantation (mean concentrations higher than 104.0 pM). These data demonstrate that islets encapsulated in C16-1A were functional in vivo, and that the hydrogel allowed diffusion of insulin into the bloodstream. Moreover, as the Wistar strain is not syngenic, these data also demonstrate the immune-protection property of the hydrogel matrix.

*d) Explants functional evaluation*

At the end of the study (28 days post implantation), the functionality of the islets within the explants C16-1A from the three rats was evaluated in vitro as described in example D2b. See Table 22.

***Table 22:*** *Functionality of encapsulated rat primary islet explants, 28 days after in vivo implantation in rat omentum. N=1 independent experiment, n=3 samples.*

| Rat ID | In vitro evaluation of islets functionality within the explants C16-1A at D28 |
|---|---|
| | GSIS Secretion Index |
| 129 | 2.1 |
| 130 | 2.2 |
| 133 | 2.1 |

Encapsulated islets displayed insulin increase in response to glucose, 28 days after implantation: indeed, GSIS secretion index superior to 2 were measured in vitro. Therefore, the functionality of rat primary islets encapsulated in C16-1A is maintained for at least 28 days after implantation in rat omentum.

*e) Explants tolerance evaluation*

Local tolerance of explants was evaluated by histology after processing and Hermatoxilyn-Eosin staining. The implant consisting of the ring net / hydrogel / allogenic islets was well tolerated, and embedded within a fibrous capsule, with no adherence to the surrounding tissues except at the level of the suture stitches. The thickness of the surrounding capsule was related to the exposure of some fibers of the net which were incompletely embedded within the gel. The gel exerted thus a protective effect against allogeneic islets rejection but also against the proinflammatory properties of the net material and structure.

**Example D5: *In vivo* survival and functionality of islets encapsulated in C16-2A**

*a) Induction of diabetes*

Diabetes was induced as described in Example D4. Animals were supplemented with a bolus administration of Lantus given in the morning, as necessary.

*b) Rat primary islet encapsulation*

Wistar rat primary islets were encapsulated 1 day after isolation, as described in example C16-2A.

*c) In vivo implantation*

In vivo implantation was conducted as described in Example D4. Lewis rats weighing approx. 250 g at surgery were used. Each animal was implanted with 4 000 IEq (at a density of 16 000 IEq/mL). An islets-free group implanted C16-2B served as a control.

Two C16-2A implants were fixed with 2 sutures onto the peritoneum, one on each side of the abdominal cavity.

After 5 weeks, the animals were sacrificed. The implantation sites were collected; one explant from each animal was dissected free from tissues for post-implantation perifusion and viability assays, and the other one processed for histology.

*d) Evolution of insulinemia*

Islet functionality was assessed by specific measurement of rat plasma insulin. Blood was collected 2 days before implantation, the day of implantation (prior to surgery) and then 28 and 35 days post implantation. Plasma was prepared and rat insulin measured using a qualified specific ELISA immunoassay (Mercodia, Ultrasensitive Rat Insulin ELISA, Ref. 10-1251-01). See Table 23.

***Table 23:*** *Insulin concentrations in plasma of diabetic rats implanted with C16-2A for 35 days. Indicated days are day post implantation. The lower limit of quantification (LLOQ) of the ELISA method was defined at 32.0 pM. <LLOQ means that insulin levels were below the LLOQ of the method.*

| Group | Rat ID | Plasmatic insulin concentration (pM) | | |
|---|---|---|---|---|
| | | Before implantation | Day 28 | Day 35 |
| Control (C16-2B) | 998 | <LLOQ | <LLOQ | <LLOQ |
| | 999 | <LLOQ | <LLOQ | <LLOQ |
| | 000 | <LLOQ | <LLOQ | <LLOQ |
| Implanted rats (C16-2A) | 975 | <LLOQ | 65.7 | 43.5 |
| | 976 | <LLOQ | 69.0 | 46.6 |
| | 977 | <LLOQ | 94.9 | 56.2 |
| | 978 | <LLOQ | 36.3 | <LLOQ |

28 and 35-days post implantation, 100% and 75% of the rats implanted with C16-2A presented detectable levels of circulating insulin, respectively, while animals treated with control hydrogels (with no encapsulated islets, C16-2B) display no detectable insulin.

These data demonstrate that encapsulated islets were functional, and that the hydrogel allowed diffusion of insulin into the bloodstream until at least 35 days post implantation. Moreover, as the Lewis islets were implanted into Wistar rats, these data also demonstrate the immune-protection property of the hydrogel matrix for at least 35 days.

*e) Explants histological evaluation*

The implants were free of any adherence within the peritoneal cavity. They were never surrounded by fibrous tissue, and no capsule was observed at histology. Islet survival observed without immune cell infiltration and no evidence of rejection.

**Example D6: *In vivo* survival and functionality of islets encapsulated in C16-3A and C16-3B**

*f) Rat primary islet encapsulation*

Rat primary islets were encapsulated 1 day after isolation, as described in example C16-3A and C16-3B and implanted in the omentum (1 implant) and the internal face of the peritonea (2 implants) of rats.

*g) In vivo implantation*

The animal was implanted in the omentum (1 implant C16-3A) and the internal face of the peritonea (2 implants C16-3B). The animal was implanted with 4 000 IEq at the density of 7 000 IEq/mL.

*h) Evolution of glycemia*

Glycemia was measured daily in a diabetic (streptozotocin treated) rat using a glucometer (Roche Guide), starting the day of implantation.

A pre-implantation blood glucose measurement was performed. Then, glycemia was taken in the morning prior to Lantus administration.

A drop of blood glucose was obtained from the vein tail

The animal implanted with C16-3A and C16-3B show a notable drop in glycemia from Day 1 post-implantation, and last until Day 97 post-implantation; values dropped from around 6g/L pre-implantation to around 2.25 g/L (1.10 g/L at the lowest), while glycemia of the other animals was not regulated and all of them were hyperglycemic (average blood glucose level of 5.3 g/L).

These data demonstrate that the encapsulated islets in the hydrogel are able to regulate glycemia, and therefore reduce glycemic excursions after a meal.

*a) Evolution of insulinemia*

Islets functionality was assessed by specific measurement of rat insulin in the plasma up to 89 days after implantation. Blood was collected 2-, 7-, 14-, 21-, 28-, 35-, 42-, 49-, 56-, 64-, 70-, 78-, 82- and 89-days post implantation. Plasma was prepared and rat insulin measured using a qualified specific ELISA immunoassay (Mercodia, Ultrasensitive Rat Insulin ELISA, Ref. 10-1251-01). See Table 24.

***Table 24:*** *Insulin concentrations in plasma of the diabetic rat implanted with C16-3A / C16-38 for 89 days. Indicated days are day post implantation. The lower limit of quantification of the ELISA method was defined at 32.0 pM.*

| Rat ID | Plasmatic insulin concentration (pM) | | | | | | |
|---|---|---|---|---|---|---|---|
| | D2 | D7 | D14 | D21 | D28 | D35 | D42 |
| 338 | 193.8 | 203.1 | 190.8 | 115.0 | 110.4 | 79.7 | 258.6 |
| | D49 | D56 | D64 | D70 | D78 | D82 | D89 |
| 338 | 187.7 | 115.8 | 192.7 | 160. 4 | 267.4 | 186.8 | 317.5 |

Stable plasmatic insulin levels were detectable from 2- to 89- days post implantation (100% of timepoints) of C16-3A / C16-3B in a diabetic rat.

These data demonstrate that encapsulated islets were functional, and that the hydrogel allowed diffusion of insulin into the bloodstream until at least 89 days post implantation. Moreover, as Lewis islets were implanted into a Wistar rat, these data also demonstrate the immune-protection property of the hydrogel matrix for at least 89 days.

**Claims**

**1.** Hydrogel comprising:

- biological cells,
- a non crosslinked hyaluronic acid in the form of a solution, and
- a crosslinked dextran polymer Dx bearing anionic groups wherein the at least divalent radical $L(-)_i$ is covalently

bound to the dextran polymer backbone with i W radicals, wherein,
- $L(-)_i$ is a linear or branched polyether
- i is the valence of L and the number of W radicals bound to the dextran polymer and is an integer comprised from 2 to 8 ($2 \leq i \leq 8$),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

**2.** Therapeutic use of the hydrogel according to claim 1, for treating a disorder or disease in a mammal wherein the disorder or disease is due to lack or malfunction of endocrine function of pancreas organ.

**3.** Implant comprising the hydrogel according to any one of claims 1 to 2.

**4.** Hydrogel according to claim 1 to 3, wherein the central-linker $L(-)_i$ of the cross-linked dextran polymer is a branched PEG radical which possesses at most 8 arms.

**5.** Hydrogel according to claim 1 to 4, wherein to claim 1, wherein the anionic groups of the cross-linked dextran polymer are chosen among alkyl carboxylate anions, sulphate anions, or sulfonate anions, or phosphate anions, or phosphonate anions.

**6.** Hydrogel according to claim 1 to 5, wherein to claim 1, wherein the anionic groups of the cross-linked dextran polymer are chosen among anionic groups of Formula II:

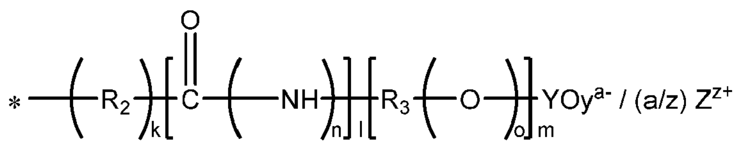

II

Wherein:

- * represents the link to the O atoms of the dextran to form an ether function.
- y=2 or 3.
- When y=2, alkyl carboxylate derivatives, then:
  - Y=C and a=1.
  - k=1, l=0 and m=0.
  - $R_2$=Alkyl.
- When y=3, anionic group, then:
  - Y=S and a=1, or Y=P and a=2.
  - k=0 or 1.
  - l=0 or 1.
  - m=0 or 1.
  - n=1 or 2, in particular n=1.
  - o=0 or 1.
  - if l=1 then m=1.
  - $R_3$=linear, branched, or cyclic alkyl which may contain one heteroatom such as nitrogen, or aromatic, or PEG.
  - $R_2$=Alkyl.
- And, Z is a counter ion, which can be an alkali metal and z=1, or which can be an alkaline earth metal and z=2.

**7.** Hydrogel according to claim 1 to 6, wherein the anionic groups of the cross-linked dextran polymer are chosen among wherein -W- is chosen among the radicals of formula IV.

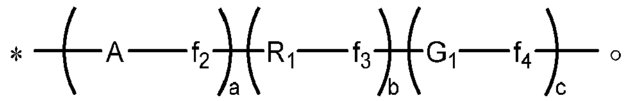

IV

Wherein

- is the site of $f_1$ and ° is the site of attachment with La is an integer equal to 0 or 1.
- b is an integer equal to 0 or 1.
- c is an integer equal to 0 or 1.
- i is an integer comprised from 2 to 8, ($2 \leq i \leq 8$).
- In one embodiment a=0, $f_1$ is an ether function, or a carbamate function.
- In one embodiment a=1,

    - the divalent radical -A- is a linear, $-(CH_2)_{n1}-$ with $n_1$ an integer comprised from 1 to 7 ($1 \leq n_1 \leq 7$), branched, or cyclic alkyl derivative. It may also be branched by at least one hydroxyl group, $-CH_2-CH(OH)-(CH_2)_{n2}-$ with $n_2$ an integer comprised from 1 to 5 ($1 \leq n_2 \leq 5$); $f_1$ is an ether function, or a carbamate function, and $f_2$ is an amide function.
        - Or,
    - the divalent radical -A- is a linear polyether (PEG) derivative; $f_1$ is an ether function, or a carbamate function, and $f_2$ is an amide function.
        - Or,
    - in another embodiment the divalent radical -A- is a 4-Alkyl-1,4-triazole derivative or a 4-PEG-1,4-triazole derivative; $f_1$ is an ether function, or a carbamate function, and $f_2$ is a carbon-nitrogen covalent bond.
        - Or,
    - in another embodiment the divalent radical -A- is a 1-Alkyl-1,4-triazole derivative or a 1-PEG-1,4-triazole derivative; $f_1$ is an ether function, or a carbamate function, and $f_2$ is a carbon-aromatic carbon covalent bond.

- The divalent radical $-R_1-$ is a linear, branched, or cyclic alkyl derivative, and/or an aromatic derivative, and/or a polyether (PEG) derivative, which can contain heteroatoms such as nitrogen, oxygen, or sulphur.

    - If b=0, then $f_1$ is an ether function, or a carbamate function.
    - If b=1, then $f_1$ is an ether function, or a carbamate function, and $f_3$ is an amide function, or an amine function, or an ether function, or a thioether function, or a carbamate function, or a carbon-nitrogen covalent bond, or a carbon-aromatic carbon covalent bond.

- The divalent radical $-G_1-$ is a linear, branched, or cyclic alkyl derivative, or an aromatic derivative, which can contain heteroatoms such as: at most 5 nitrogen atoms, at most 10 oxygen atoms, at most 5 sulphur atoms, or at most one phosphorus atom. In a preferred embodiment, $-G_1-$ is a succinimide derivative, or an alkyl sulfone derivative which can contain one heteroatom such as oxygen or sulphur, or an ethyl amide derivative, or a 1,4-triazole derivative, or a multicycle derivative from a Diels-Alder reaction, or an aromatic phosphine derivative created by a Staudinger ligation, or a cysteine derivative coming from a Native Chemical Ligation.

    - If c=0, then $f_1$, is an ether function, or a carbamate function.
    - If c=1, then $f_1$, is an ether function, or a carbamate function, and $f_4$ is an amine function, or an amide function, or a carbamate function, or a thioether function, or an ether function, or a carbon-nitrogen covalent bond, or carbon-aromatic carbon covalent bond.

**8.** Kit comprising :

- A solution of dextran polymer of formula VIII before the crosslinking reaction:

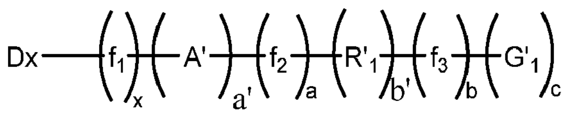

VIII

Wherein

- $f_1$, $f_2$, $f_3$, $f_4$, Dx are defined as in formula IV if none of a, a', b and b' are not equal to 0,
- and
- x equal 0 or 1
- if a, a', b and b' are equal to 0, x is equal to 0 and Dx is a dextran polymer backbone is according to formula III, wherein R is chosen among -H or a anionic group of formula II,
- if one of b' and c is not equal to 0 -A' is A as defined above,
- if b', b and c are equal to 0, a is equal to 0 and A' is the precursor of A before the crosslinking reaction.
- if c is not equal to 0, $R'_1$ is $R_1$ as defined above and $G'_1$ is the precursor of $G_1$.
- if c is equal to 0, b is equal to 0 and $R'_1$ is the precursor of $R_1$ before the crosslinking , reaction.

- a solution of a thiol polyethylene glycol, a mercaptopoly(oxyethylenes), a pentaerythritol poly(oxyethylene) azide or a pentaerythritol poly(dibenzocyclooctyne) polyoxyethylene.
- biological cells,
- a solution of non crosslinked sodium hyaluronate.

**9.** Process to synthetize a hydrogel according claim 1 to 7 comprising the steps of:

a) preparation of a sterile solution comprising a dextran bearing anionic groups of formula II and at least two precursors of -W-
b) preparation of a sterile solution of a precursor of $L(-)_i$ chosen among a thiol polyethylene glycol, a mercaptopoly(oxyethylenes), a pentaerythritol poly(oxyethylene) azide or a pentaerythritol poly(dibenzocyclooctyne) polyoxyethylene
c) preparation of a sterile solution of hyaluronate de sodium,
d) preparation of a sterile suspension of biological cells,
e) mixing the sodium hyaluronate solution obtained from step c) with precursor solution from the step b)
f) mixing the biological cells suspension obtained from step d and the solution obtained from the step e) or from step a)
g) mixing the solution obtained from step f) and the solutions obtained from step e),
h) addition of the sterile solution obtained from step a) or e) which is not used in step g) to the solution obtained from step f),
i) the addition of step g) being either done directly in a mould or the solutions are introduced into a mould after being mixed,
j) cross-linking and gelation reaction at room temperature (20-25°C), unmoulding and swelling to obtain an hydrogel comprising biological cells.

**10.** Process according to claim 10, wherein the mould is a Ring Net.

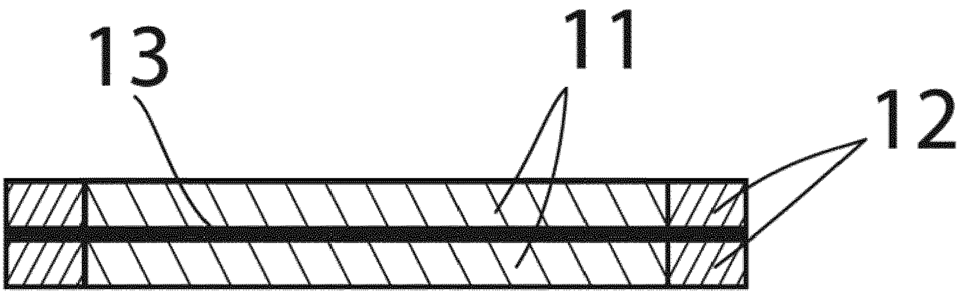


FIGURE 1

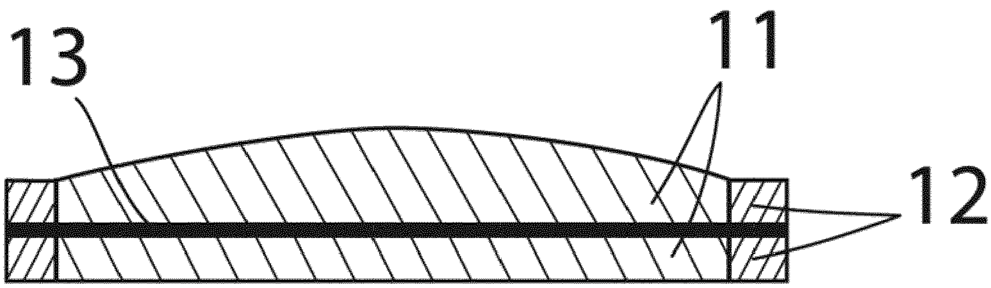


FIGURE 2

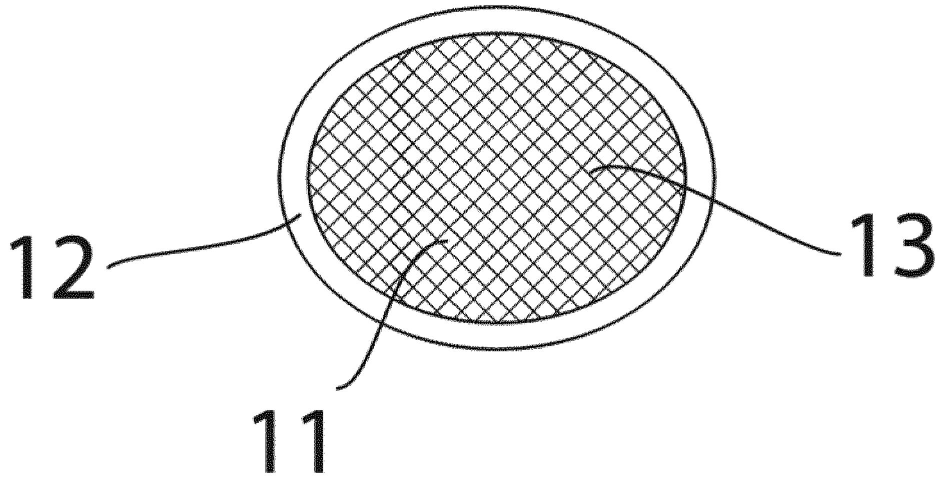


FIGURE 3

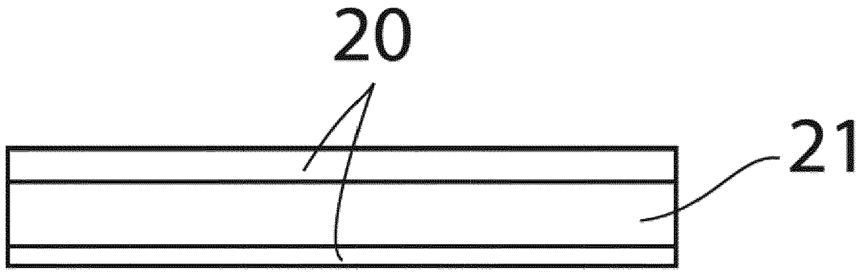


FIGURE 4

Europäisches Patentamt
European Patent Office
Office européen des brevets

# EUROPEAN SEARCH REPORT

Application Number
EP 22 18 4567

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 2 716 662 A1 (UNIV TOKYO [JP]) 9 April 2014 (2014-04-09) * paragraphs [0001], [0007], [0019], [0041] - [0042], [0056] * * examples 1-5, 7-11, 13-15 * * claims 20-24 * ----- | 1-10 | INV. C08B37/02 A61K47/36 A61L27/52 C08J3/075 C08L5/02 C08B37/08 |
| X | TRUDEL J ET AL: "Assessment of the cytotoxicity of photocrosslinked dextran and hyaluronan-based hydrogels to vascular smooth muscle cells", BIOMATERIALS, ELSEVIER, AMSTERDAM, NL, vol. 23, no. 16, 1 August 2002 (2002-08-01), pages 3299-3307, XP004359617, ISSN: 0142-9612, DOI: 10.1016/S0142-9612(02)00009-1 * abstract * * page 3300, paragraphs 2., 2.1.1, 2.1.3, 2.1.4, 2.5 - page 3302 * ----- | 1-10 | |
| | | | TECHNICAL FIELDS SEARCHED (IPC) |
| | | | C08B A61K C08J A61L C08L |

The present search report has been drawn up for all claims

1

| Place of search | Date of completion of the search | Examiner |
|---|---|---|
| Munich | 20 December 2022 | Lartigue, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 22 18 4567

This annex lists the patent family members relating to the patent documents cited in the above-mentioned European search report. The members are as contained in the European Patent Office EDP file on The European Patent Office is in no way liable for these particulars which are merely given for the purpose of information.

20-12-2022

| Patent document cited in search report | | Publication date | Patent family member(s) | | Publication date |
|---|---|---|---|---|---|
| EP 2716662 | A1 | 09-04-2014 | EP 2716662 | A1 | 09-04-2014 |
| | | | JP WO2012165462 | A1 | 23-02-2015 |
| | | | US 2014256831 | A1 | 11-09-2014 |
| | | | WO 2012165462 | A1 | 06-12-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2022050466 W


### Non-patent literature cited in the description


- **NESTOR LOPER MORA et al.** evaluation of dextran(ethyleneglycol) hydrogel films for giant unilamellar lipid vesicle production and their application for the encapsulation of polymersome. *Soft Matters,* January 2017, vol. 13 (33), 5580-5585
- **HANWEI ZHANG et al.** In situ gelable interpenetrating double network hydrogel formulated from binary components: thiolated chitosan and oxidized dextran. *Biomacromolecules,* 2011, vol. 12 (5), 1428-1437
- **RONGSHENG ZHANG et al.** A novel pH and ionic strength sensitive carboxymethyl dextran hydrogsel. *Biomaterials,* 2005, vol. 26 (22), 4677-4683
- **TAICHI ITO et al.** Dextran-based in situ cross-linked injectable hydrogels to prevent peritoneal adhesions. *Biomaterials,* 2007, vol. 28 (23), 3418-3426
- *CHEMICAL ABSTRACTS,* 188492-68-4
- *CHEMICAL ABSTRACTS,* 68865-60-1
- **S. A. STEWART et al.** *Soft Matter,* 2018, vol. 14, 8317
- **L. YANG et al.** *J. Mater. Chem. B,* 2013, vol. 1, 1421
- **CARTER et al.** A Pratical Guide to Rodent Islet Isolation and Assessment. *Biological Procedures Online,* 2009
- Polysaccharide Hydrogels: Characterization and Biomedical Applications. Pan Stanford Publishing Pte. Ltd, 2016, 97